(19) Europäisches Patentamt
European Patent Office
Office européen des brevets

(11) Veröffentlichungsnummer: **0 520 240 A1**

(12) # EUROPÄISCHE PATENTANMELDUNG

(21) Anmeldenummer: **92109742.4**

(22) Anmeldetag: **10.06.92**

(51) Int. Cl.5: **C07D 401/04**, C07D 209/44, C07D 215/56, A61K 31/47

(30) Priorität: **22.06.91 DE 4120646**

(43) Veröffentlichungstag der Anmeldung: **30.12.92 Patentblatt 92/53**

(84) Benannte Vertragsstaaten: **AT BE CH DE DK ES FR GB GR IT LI LU NL PT SE**

(71) Anmelder: **BAYER AG**

**W-5090 Leverkusen 1 Bayerwerk(DE)**

(72) Erfinder: **Petersen, Uwe, Dr.**
**Auf dem Forst 4**
**W-5090 Leverkusen 1(DE)**
Erfinder: **Krebs, Andreas, Dr.**
**Am Gartenfeld 70**
**W-5068 Odenthal(DE)**
Erfinder: **Schenke, Thomas, Dr.**
**Mühlenstrasse 113**
**W-5060 Bergisch Gladbach(DE)**
Erfinder: **Kunisch, Franz, Dr.**
**Zum Hahnenberg 20**
**W-5068 Odenthal-Glöbusch(DE)**
Erfinder: **Philipps, Thomas, Dr.**
**Silesiusstrasse 74**
**W-5000 Köln 80(DE)**
Erfinder: **Grohe, Klaus, Dr.**
**Am Wasserturm 10**
**W-5068 Odenthal(DE)**
Erfinder: **Bremm, Klaus-Dieter, Dr.**
**Giradet-Strasse 120**
**W-5600 Wuppertal(DE)**
Erfinder: **Endermann, Rainer, Dr.**
**In den Birken 152 a**
**W-5600 Wuppertal(DE)**
Erfinder: **Metzger, Karl Georg**
**Pahlkestrasse 75**
**W-5600 Wuppertal(DE)**
Erfinder: **Haller, Ingo, Dr.**
**Dornröschenweg 4**
**W-5600 Wuppertal 1(DE)**
Erfinder: **Zeiler, Hans-Joachim, Dr.**
**Am Rohm 86**
**W-5600 Wuppertal(DE)**

(54) **7-Isoindolinyl-chinolon- und naphthyridoncarbonsäure-Derivate.**

(57) Die Erfindung betrifft neue Chinolon- und Naphthyridoncarbonsäure-Derivate, die in 7-Stellung durch einen partiell hydrierten Isoindolinylring substituiert sind, Verfahren zu ihrer Herstellung sowie diese enthaltende antibakterielle Mittel und Futterzusatzstoffe.

EP 0 520 240 A1

Die Erfindung betrifft neue Chinolon- und Naphthyridoncarbonsäure-Derivate, die in 7-Stellung durch einen partiell hydrierten Isoindolinylring substituiert sind, Verfahren zu ihrer Herstellung sowie diese enthaltende antibakterielle Mittel und Futterzusatzstoffe.

Es sind bereits aus der EP 343 560 Chinolon- und Naphthyridoncarbonsäuren bekannt geworden, die in 7-Stellung durch einen Isoindolinylring substituiert sind, wie z.B. 7-(2-Isoindolinyl)-1-cyclopropyl-6,8-difluor-1,4-dihydro-4-oxo-3-chinolincarbonsäure. Diese zeichnen sich jedoch nur durch eine geringe antibakterielle Wirksamkeit aus.

Es wurde gefunden, daß die Verbindungen der Formel (I)

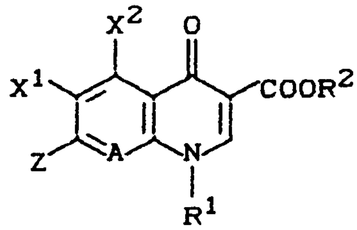

(I),

in welcher

$X^1$ für Halogen,

$X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,

$R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,

$R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,

Z für einen Rest der Struktur

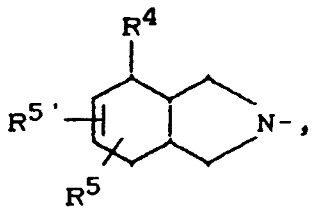

steht, worin

$R^4$ für Wasserstoff, Hydroxy,

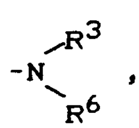

Hydroxymethyl oder

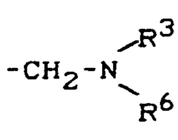

steht, wobei

$R^3$ Wasserstoff, gegebenenfalls durch Hydroxy substituiertes $C_1$-$C_3$-Alkyl, Alkoxycarbonyl mit 1 bis 4

C-Atomen im Alkoxyteil oder $C_1$-$C_3$-Acyl und

$R^6$ Wasserstoff oder Methyl bedeutet,

$R^5$ für Wasserstoff, $C_1$-$C_3$-Alkyl oder Cyclopropyl,

$R^{5'}$ für Wasserstoff oder Methyl und

A für N oder C-$R^7$ steht, worin

$R^7$ für H, Halogen, Methyl, Hydroxy oder Methoxy steht oder auch gemeinsam mit $R^1$ eine Brücke der Struktur

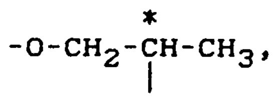, 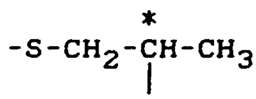 oder

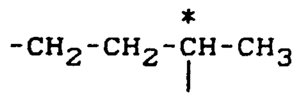

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren gegenüber dem Stand der Technik eine höhere antibakterielle Wirkung insbesondere im grampositiven Bereich aufweisen.

Bevorzugt sind die Verbindungen der Formel (I),
in welcher

$X^1$ für Fluor,

$X^2$ für Wasserstoff, Amino, Methylamino, Hydroxy, Methoxy, Fluor, Chlor, Brom oder Methyl,

$R^1$ für Alkyl mit 1 bis 3 Kohlenstoffatomen, Vinyl, Cycloalkyl mit 3 bis 4 Kohlenstoffatomen, 2-Fluorethyl, gegebenenfalls durch 1 oder 2 Fluoratome substituiertes Phenyl,

$R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,

Z für einen Rest der Struktur

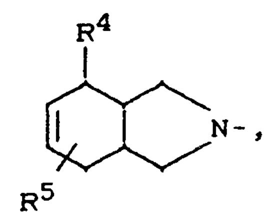

steht, worin

$R^4$ für Wasserstoff, Hydroxy oder

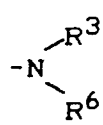

steht, wobei

$R^3$ Wasserstoff, gegebenenfalls durch Hydroxy substituiertes $C_1$-$C_2$-Alkyl, Alkoxycarbonyl mit 1 bis 4 C-Atomen im Alkoxyteil oder $C_1$-$C_3$-Acyl und

$R^6$ Wasserstoff oder Methyl bedeutet,

$R^5$ für Wasserstoff oder Methyl und

A für N oder C-$R^7$ steht, worin

$R^7$ für H, Fluor, Chlor, Brom, Methyl oder Methoxy steht oder auch gemeinsam mit $R^1$ eine Brücke der Struktur

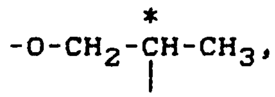

bilden kann
und deren pharmazeutisch verwendbare Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I), in welcher

$X^1$ für Fluor,
$X^2$ für Wasserstoff, Amino, Fluor, Chlor oder Brom,
$R^1$ für Alkyl mit 1 bis 2 Kohlenstoffatomen, Cyclopropyl, gegebenenfalls durch 1 oder 2 Fluoratome substituiertes Phenyl,
$R^2$ für Wasserstoff oder Alkyl mit 1 bis 2 Kohlenstoffatomen,
Z für einen Rest der Struktur

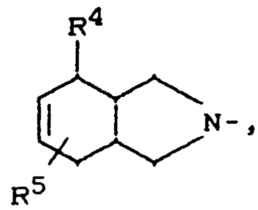

steht, worin
$R^4$ für Wasserstoff, Hydroxy oder

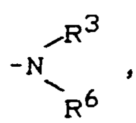

steht, wobei
$R^3$ Wasserstoff, Methyl, Alkoxycarbonyl mit 1 bis 4 C-Atomen im Alkoxyteil oder $C_1$-$C_3$-Acyl und
$R^6$ Wasserstoff oder Methyl bedeutet,
$R^5$ für Wasserstoff oder Methyl und
A für N oder C-$R^7$ steht, worin
$R^7$ für H, Fluor, Chlor oder Methoxy steht oder auch gemeinsam mit $R^1$ eine Brücke der Struktur

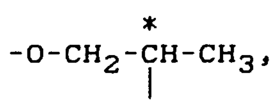

bilden kann,
und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man eine Verbindung der Formel (II)

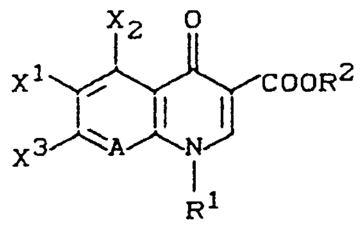

(II),

in welcher

A, $R^1$, $R^2$, $X^1$ und $X^2$ die oben angegebene Bedeutung haben und

$X^3$ für Halogen, insbesondere Fluor oder Chlor steht,

mit Verbindungen der Formel (III)

Z-H (III)

in welcher

Z die oben angegebene Bedeutung hat,

gegebenenfalls in Gegenwart von Säurefängern umsetzt.

Verwendet man beispielsweise 1-Cyclopropyl-6,7,8-trifluor-1,4-dihydro-4-oxo-3-chinolincarbonsäure und cis-7-Methyl-4-methylamino-1,3,3a,4,7,7a-hexahydro-isoindol als Ausgangsstoffe, so kann der Reaktionsverlauf durch folgendes Formelschema wiedergegeben werden:

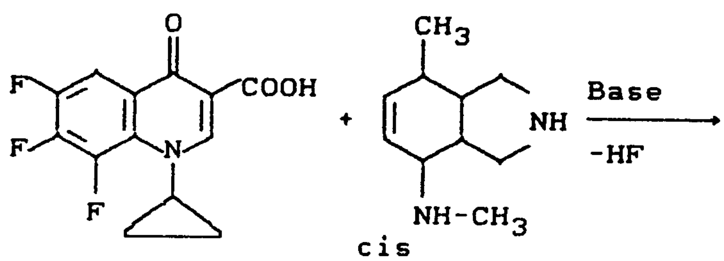


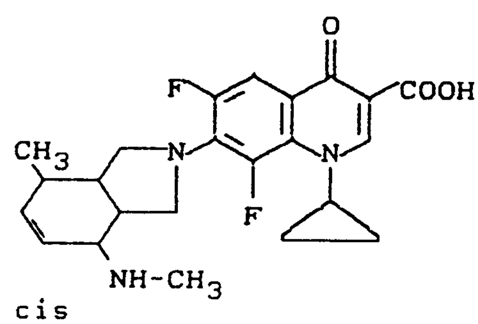

Verwendet man beispielsweise 7-Chlor-6-fluor-1-(4-fluorphenyl)-1,4-dihydro-4-oxo-1,8-naphthyridin-3-carbonsäureethylester und 4-Methylamino-1,3,3a,4,7,7a-hexahydroisoindol als Ausgangsstoffe, so kann der Reaktionsverlauf durch das folgende Formelschema wiedergegeben werden:

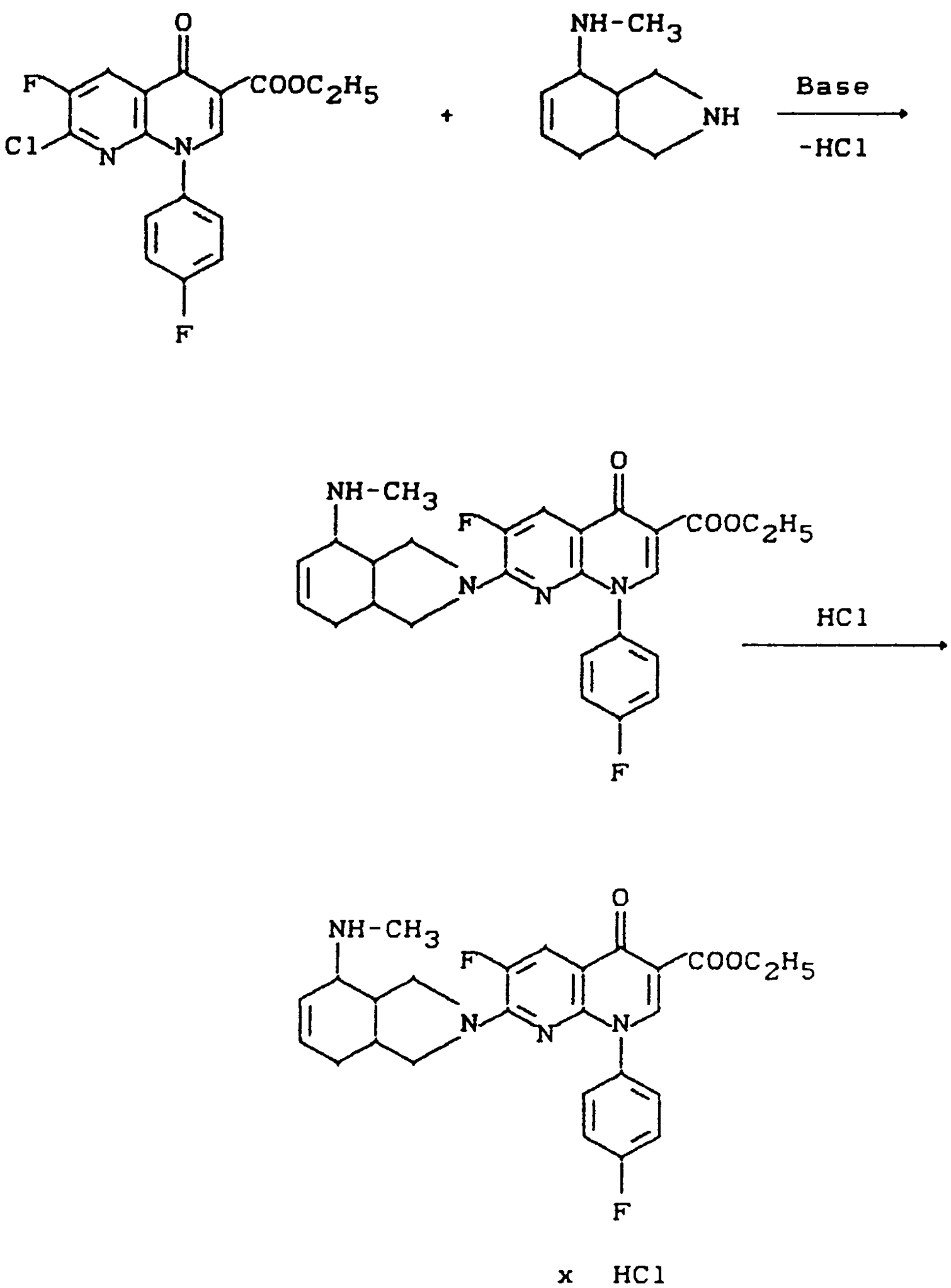


Verwendet man beispielsweise 1-Cyclopropyl-5,6,8-trifluor-1,4-dihydro-7-(4-methylamino-1,3,3a,4,7,7a-hexahydro-isoindol-2-yl)-4-oxo-3-chinolincarbonsäure und Ammoniak als Ausgangsstoffe, so kann der Reaktionsablauf durch folgendes Formelschema wiedergegeben werden:

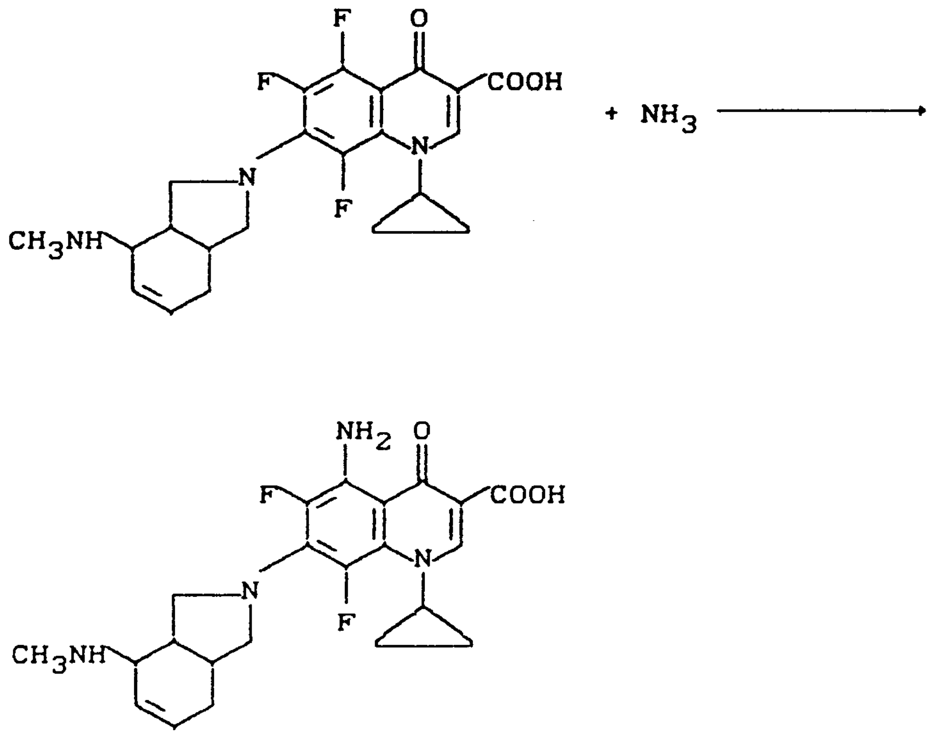


Verwendet man beispielsweise 1-Cyclopropyl-6-fluor-1,4-dihydro-7-(cis-7-methyl-4-methylamino-hexahydroisoindol-2-yl)-4-oxo-3-chinolincarbonsäure und Ethanol/Chlorwasserstoff als Ausgangsstoffe, so kann der Reaktionsverlauf durch folgendes Formelschema wiedergegeben werden:

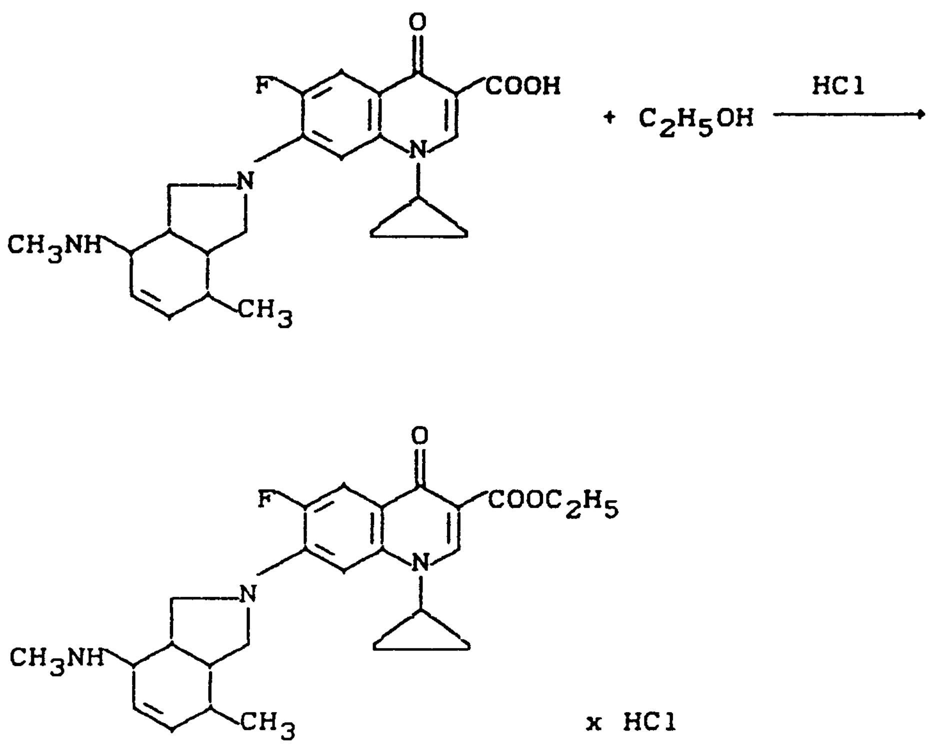


Die meisten der als Ausgangsstoffe verwendeten Verbindungen der Formel (II) sind bekannt oder können nach bekannten Methoden hergestellt werden. Als Beispiele seien genannt:

7-Chlor-1-cyclopropyl-6-fluor-1,4-dihydro-4-oxo-3-chinolincarbonsäure (Deutsche Patentanmeldung 3 142 854),

1-Cyclopropyl-6,7-difluor-1,4-dihydro-4-oxo-3-chinolincarbonsäure (Europäische Patentanmeldung 113 091),
6-Chlor-1-cyclopropyl-7,8-difluor-1,4-dihydro-4-oxo-3-chinolincarbonsäure (Deutsche Patentanmeldung 3 420 743),
8-Chlor-1-cyclopropyl-6,7-difluor-1,4-dihydro-4-oxo-3-chinolincarbonsäure (Deutsche Patentanmeldung 3 420 743),
1-Cyclopropyl-6,7,8-trifluor-1,4-dihydro-4-oxo-3-chinolincarbonsäure (Deutsche Patentanmeldung 3 318 145),
5-Brom-1-cyclopropyl-6,7,8-trifluor-1,4-dihydro-4-oxo-3-chinolincarbonsäure,
5-Brom-1-(2,4-difluorphenyl)-6,7,8-trifluor-1,4-dihydro-4-oxo-3-chinolincarbonsäure,
1-Cyclopropyl-6,7-difluor-1,4-dihydro-8-methyl-4-oxo-3-chinolincarbonsäure,
6,7-Difluor-1-ethyl-1,4-dihydro-4-oxo-3-chinolincarbonsäure,
7-Chlor-6-fluor-1-ethyl-1,4-dihydro-4-oxo-3-chinolincarbonsäure,
7-Chlor-6-fluor-1,4-dihydro-1-(2-hydroxyethyl)-4-oxo-3-chinolincarbonsäure,
6,7-Difluor-1-(2-fluorethyl)-1,4-dihydro-4-oxo-3-chinolincarbonsäure,
7-Chlor-6-fluor-1,4-dihydro-1-methoxy-4-oxo-3-chinolincarbonsäure,
7-Chlor-6-fluor-1,4-dihydro-1-methylamino-4-oxo-3-chinolincarbonsäure,
6,7-Difluor-1,4-dihydro-4-oxo-1-phenyl-3-chinolincarbonsäure,
7-Chlor-1-cyclopropyl-6-fluor-1,4-dihydro-4-oxo-1,8-naphthyridin-3-carbonsäure,
7-Chlor-1-cyclopropyl-6-fluor-1,4-dihydro-4-oxo-1,8-naphthyridin-3-carbonsäureethylester,
1-Cyclopropyl-6,7,8-trifluor-1,4-dihydro-4-oxo-3-chinolincarbonsäureethylester (Deutsche Patentanmeldung 3 318 145),
9,10-Difluor-2,3-dihydro-3-methyl-7-oxo-7H-pyrido-[1,2,3-de][1,4]benzoxacin-6-carbonsäure (Europäische Patentanmeldung 47 005),
8,9-Difluor-6,7-dihydro-5-methyl-1-oxo-1H,5H-benzo[i,j]chinolicin-2-carbonsäure,
7-Chlor-6-fluor-1-phenyl-1,4-dihydro-4-oxo-1,8-naphthyridin-3-carbonsäure (Europäische Patentanmeldung 153 580),
7-Chlor-6-fluor-1-(4-fluorphenyl)-1,4-dihydro-4-oxo-1,8-naphthydrin-3-carbonsäureethylester,
6,7,8-Trifluor-1,4-dihydro-1-methylamino-4-oxo-3-chinolincarbonsäure (Deutsche Patentanmeldung 3 409 922),
1-Amino-6,7,8-trifluor-1,4-dihydro-4-oxo-3-chinolincarbonsäure (Deutsche Patentanmeldung 3 409 922),
6,7,8-Trifluor-1,4-dihydro-1-dimethylamino-4-oxo-3-chinolincarbonsäure (Deutsche Patentanmeldung 3 409 922),
6,7-Difluor-1-(4-fluorphenyl)-1,4-dihydro-8-methyl-4-oxo-3-chinolincarbonsäure,
7-Chlor-6-fluor-1-(4-fluorphenyl)-1,4-dihydro-4-oxo-3-chinolincarbonsäure (Europäische Patentanmeldung 131 839),
7-Chlor-6-fluor-1-(2,4-difluorphenyl)-1,4-dihydro-4-oxo-3-chinolincarbonsäure (Europäische Patentanmeldung 131 839),
6,7,8-Trifluor-1-(4-fluorphenyl)-1,4-dihydro-4-oxo-3-chinolincarbonsäure (Europäische Patentanmeldung 154 780),
6,7,8-Trifluor-1-(2,4-difluorphenyl)-1,4-dihydro-4-oxo-3-chinolincarbonsäure (Europäische Patentanmeldung 154 780),
6,7,8-Trifluor-1,4-dihydro-4-oxo-1-phenyl-3-chinolincarbonsäure (Europäische Patentanmeldung 154 780),
7-Chlor-1-ethyl-6-fluor-1,4-dihydro-4-oxo-1,8-naphthyridin-3-carbonsäure,
6,7-Difluor-1,4-dihydro-4-oxo-1-vinyl-3-chinolincarbonsäure,
1-Cyclopropyl-5,6,7,8-tetrafluor-1,4-dihydro-4-oxo-3-chinolincarbonsäure,
5-Amino-1-cyclopropyl-6,7,8-trifluor-1,4-dihydro-4-oxo-3-chinolincarbonsäure,
1-Cyclopropyl-6,7,8-trifluor-1,4-dihydro-5-hydroxy-4-oxo-3-chinolincarbonsäure,
1-Cyclopropyl-6,7-difluor-1,4-dihydro-8-methoxy-4-oxo-3-chinolincarbonsäure,
7-Chlor-1-(2,4-difluorphenyl)-6-fluor-1,4-dihydro-4-oxo-1,8-naphthyridin-3-carbonsäure,
7-Chlor-1-(2,4-difluorphenyl)-6-fluor-1,4-dihydro-4-oxo-1,8-naphthyridin-3-carbonsäureethylester.

Die substituierten 1,3,3a,4,7,7a-Hexahydro-isoindole der Formel (III) sind überwiegend neu. Sie können zum Beispiel durch Diels-Alder-Reaktion von Dienen der Formel (1)

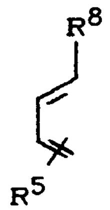

(1)

wobei $R^5$ die oben angegebene Bedeutung hat und $R^8$ entweder mit $R^4$ identisch ist oder eine funktionelle Gruppe darstellt, die in $R^4$ umgewandelt werden kann, mit Dienophilen der Formel (2),

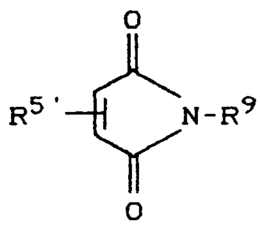

(2)

in welcher $R^9$ Wasserstoff oder eine Schutzgruppe wie Trimethylsilyl, Benzyl, $C_1$-$C_4$-Alkylphenylmethyl, Methoxy-benzyl, Benzylhydryl und $R^{5'}$ Wasserstoff oder Methyl bedeuten, und der nachfolgenden Reduktion der Carbonylgruppen sowie gegebenenfalls der Abspaltung der Schutzgruppe erhalten werden.

Für die Diels-Alder-Reaktion kommen als Verdünnungsmittel alle inerten organischen Lösungsmittel in Frage. Hierzu gehören vorzugsweise Ether, wie Diisopropylether, Di-n-butylether, Dimethoxyethan, Tetrahydrofuran und Anisol, Kohlenwasserstoffe, wie z.B. Hexan, Methylcyclohexan, Toluol, Xylol und Mesitylen und halogenierte Kohlenwasserstoffe, wie z.B. Chloroform, 1,2-Dichlorethan und Chlorbenzol. Die Diels-Alder-Reaktion kann aber auch ohne Lösungsmittel durchgeführt werden.

Die Reaktionstemperaturen können in einem größeren Bereich variiert werden. Im allgemeinen arbeitet man zwischen etwa -20° C und +200° C, vorzugsweise zwischen -20° C und +150° C. Die Diels-Alder-Reaktion wird normalerweise bei Normaldruck durchgeführt. Zur Beschleunigung der Reaktion können aber auch Drucke bis zu 1,5 GPa eingesetzt werden.

Die Reduktion der Carbonylgruppen kann mit komplexen Hydriden erreicht werden. Als Hydride können z.B. Lithiumaluminiumhydrid, Lithiumborhydrid, Lithiumtriethylborhydrid, Natrium-bis-[2-methoxyethoxy]-aluminiumhydrid oder Natriumborhydrid in Gegenwart von Lewis-Säure-Katalysatoren wie Chlortrimethylsilan, Bortrifluorid-Etherat oder Aluminiumchlorid eingesetzt werden.

Als Verdünnungsmittel können die für solche Reduktionen üblichen Lösungsmittel verwendet werden. Hierzu gehören vorzugsweise Ether, wie z.B. Diethylether, Tetrahydrofuran, Dioxan oder Dimethoxyethan und Kohlenwasserstoffe, wie z.B. Hexan, Methylcyclohexan und Toluol oder auch deren Gemische.

Die Reaktionstemperaturen können im Bereich zwischen -40 und +180° C, vorzugsweise zwischen 0° und 140° C, variiert werden. Die Reduktion wird im allgemeinen unter Normaldruck durchgeführt, sie kann aber auch unter vermindertem Druck oder unter Überdruck durchgeführt werden.

Die Anwendung von Drucken zwischen 100 und 1.000 kPa empfiehlt sich, um mit leicht siedenden Lösungsmitteln höhere Reaktionstemperaturen zu erreichen.

Die komplexen Hydride werden mindestens in einer der Stöchiometrie der Reduktion entsprechenden Menge eingesetzt. Im allgemeinen wird aber ein Überschuß, vorzugsweise zwischen 30 und 300 %, eingesetzt.

Die Abspaltung einer eventuellen Schutzgruppe erfolgt nach den allgemein bekannten Methoden der Schutzgruppenchemie (vergl. z.B. T.W. Greene, "Protective Groups in Organic Synthesis", John Wiley & Sons, New York 1981).

Die Ausgangsstoffe der Formel (1) und (2) sind bekannt oder können nach allgemein bekannten Methoden der organischen Chemie hergestellt werden [vergl. z.B. J. Am. Chem. Soc. 100, 5179 (1978), J. Org. Chem. 43, 2164 (1978), DE 39 27 115, J. Org. Chem. 40, 24 (1975)].

Verwendet man beispielsweise 1-(tert.-Butyloxycarbonylamino)-1,3-butadien und Maleinimid als Ausgangsmaterialien und Lithiumaluminiumhydrid als Reduktionsmittel, so kann der Reaktionsablauf durch das folgende Formelschema wiedergegeben werden:

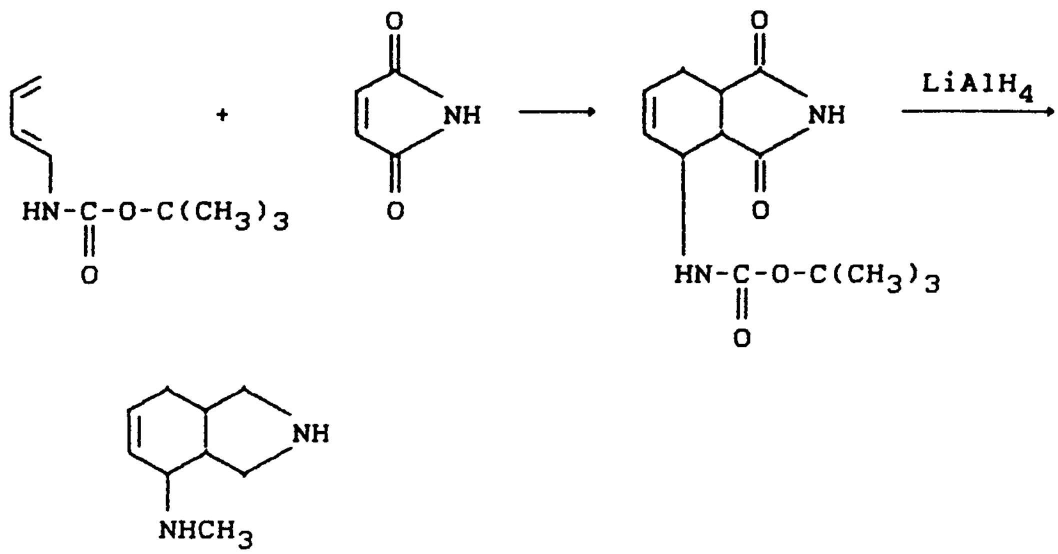


In einer besonderen Ausführungsform des Herstellungsverfahrens können alle Stufen bei Verwendung eines geeigneten Lösungsmittels, wie z.B. Tetrahydrofuran, ohne Isolierung der Zwischenprodukte durchgeführt werden. Verwendet man z.B. 1-(tert.-Butyloxycarbonylamino)-1,3-pentadien und N-Trimethylsilyl-maleinimid als Ausgangsmaterialien, so kann der Reaktionsablauf durch das folgende Formelschema wiedergegeben werden:

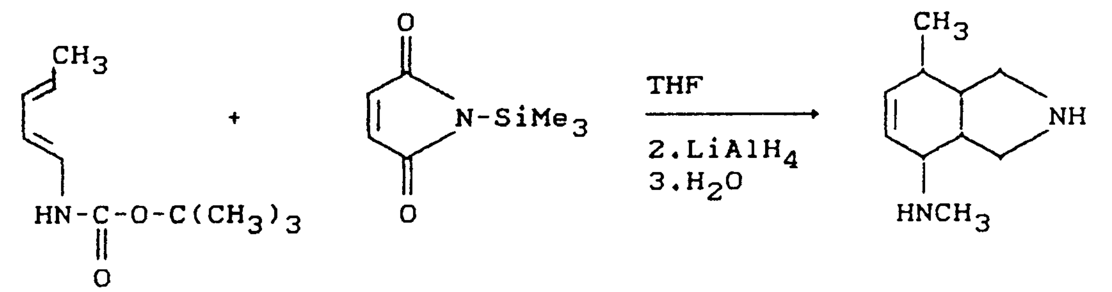


Durch NMR-Spektroskopie kann in diesem Fall nachgewiesen werden, daß alle Substituenten am 6-Ring cis-Anordnung zueinander aufweisen.

Als Beispiele für Verbindungen der Formel (III), die sowohl als Racemate als auch als enantiomeren- oder diastereomerenreine Verbindungen eingesetzt werden können, seien genannt:
4-Amino-1,3,3a,4,7,7a-hexahydroisoindol,
4-Methylamino-1,3,3a,4,7,7a-hexahydroisoindol,
5-Methyl-4-methylamino-1,3,3a,4,7,7a-hexahydroisoindol,
6-Methyl-4-methylamino-1,3,3a,4,7,7a-hexahydroisoindol,
7-Methyl-4-methylamino-1,3,3a,4,7,7a-hexahydroisoindol,
7a-Methyl-4-methylamino-1,3,3a,4,7,7a-hexahydroisoindol,
6,7-Dimethyl-4-methylamino-1,3,3a,4,7,7a-hexahydroisoindol,
7-Ethyl-4-methylamino-1,3,3a,4,7,7a-hexahydroisoindol,
7-Isopropyl-4-methylamino-1,3,3a,4,7,7a-hexahydroisoindol,
4-Methylamino-7-cyclopropyl-1,3,3a,4,7,7a-hexahydroisoindol,
4-Dimethylamino-1,3,3a,4,7,7a-hexahydroisoindol,
4-Ethylamino-1,3,3a,4,7,7a-hexahydroisoindol,
4-[(2-Hydroxyethyl)-amino]-1,3,3a,4,7,7a-hexahydroisoindol,
4-[N-(2-Hydroxyethyl)-N-methyl-amino]-1,3,3a,4,7,7a-hexahydroisoindol,
4-Aminomethyl-1,3,3a,4,7,7a-hexahydroisoindol,
4-Methylaminomethyl-1,3,3a,4,7,7a-hexahydroisoindol,
4-Hydroxy-1,3,3a,4,7,7a-hexahydroisoindol,
4-Hydroxymethyl-1,3,3a,4,7,7a-hexahydroisoindol.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200° C, vorzugsweise zwischen 80 und 180° C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol, der Verbindung (III) ein.

Freie Aminogruppen können während der Umsetzung durch eine geeignete Aminoschutzgruppe, z.B. durch den tert.-Butoxycarbonylrest oder als Azomethingruppe, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. McOmie, Protective Groups in Organic Chemistry (1973), Seite 43).

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20 bis 200° C, vorzugsweise etwa 60 bis 120° C, umgesetzt. Das entstehende Reaktionswasser kann auch durch azeotrope Destillation mit Chlorofom, Tetrachlormethan, Benzol oder Toluol entfernt werden.

Die Herstellung von Estern gelingt auch vorteilhaft durch Erhitzen der zugrundeliegenden Säure mit Dimethylformamiddialkylacetat in einem Lösungsmittel wie Dimethylformamid.

Die als Prodrug verwendeten (5-Methyl-2-oxo-1,3-dioxol-4-yl-methyl)-ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit 4-Brommethyl- oder 4-Chlormethyl-5-methyl-1,3-dioxol-2-on in einem Lösungsmittel wie Dimethylformamid, Dimethylacetamid, N-Methylpyrrolidon, Dimethylsulfoxid oder Tetramethylharnstoff bei Temperaturen von etwa 0 bis 100° C, vorzugsweise 0 bis 50° C, erhalten.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die in der folgenden Tabelle aufgeführten Wirkstoffe hergestellt werden:

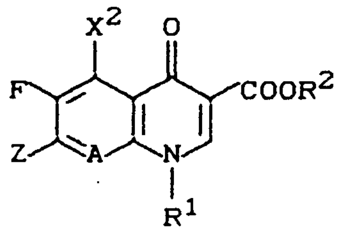

| $R^1$ | $R^2$ | $X^2$ | Z | A |
|---|---|---|---|---|
| 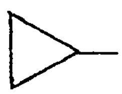 | H | H | 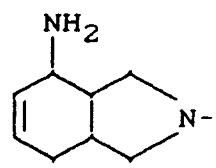 | N |
| 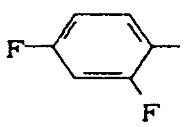 | H | H | 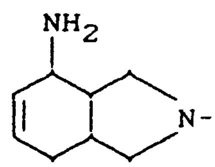 | N |
| 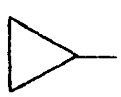 | H | Cl | 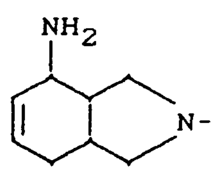 | C-Cl |

| $R^1$ | $R^2$ | $X^2$ | Z | A |
|---|---|---|---|---|
| 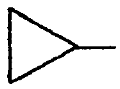 | H | H | 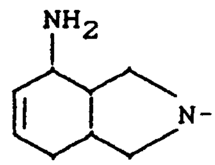 | C-$OCH_3$ |
| 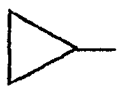 | H | H | 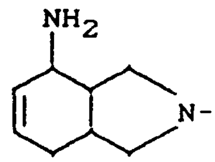 | C-$CH_3$ |
| 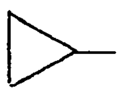 | H | $CH_3$ | 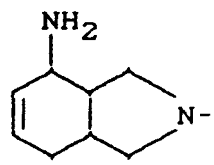 | CH |
| 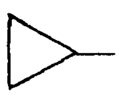 | H | $CH_3$ | 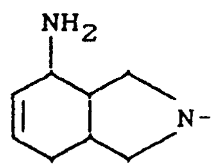 | CF |
| 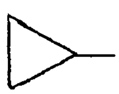 | H | H | 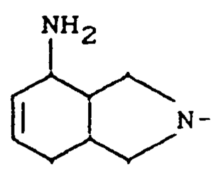 | C-Br |
| 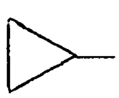 | H | Br | 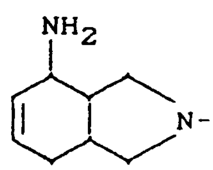 | CF |
| 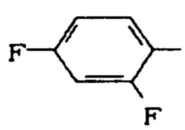 | H | Br | 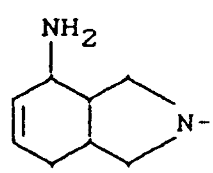 | CF |

| $R^1$ | $R^2$ | $X^2$ | Z | A |
|---|---|---|---|---|
| 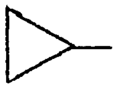 | H | F | 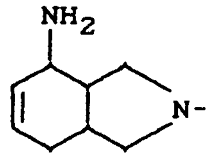 | CF |
| 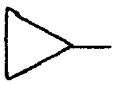 | H | F | 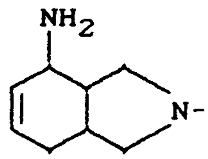 | CH |
| 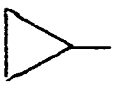 | $C_2H_5$ | H | 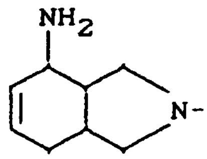 | CF |
| 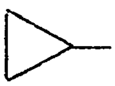 | $CH_2CH_2NH_2$ | H | 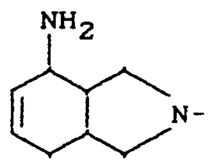 | CF |
| 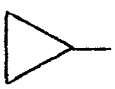 | $CH_2CH_2$-OH | H | 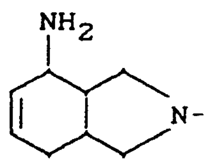 | CCl |
| $C_2H_5$ | H | H | 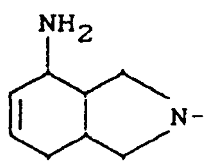 | CF |
| 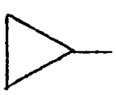 | H | $NH_2$ | 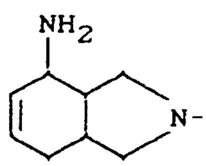 | CF |
| 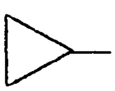 | H | H | 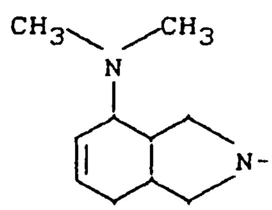 | CF |

| $R^1$ | $R^2$ | $X^2$ | Z | A |
| --- | --- | --- | --- | --- |
| 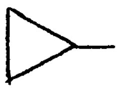 | H | H | 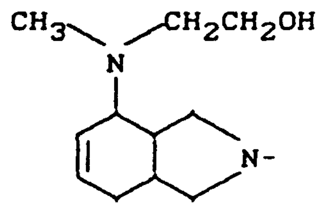 | CF |
| 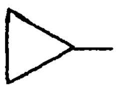 | H | H | 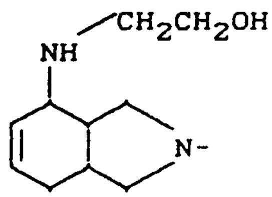 | CF |
| 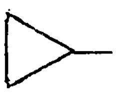 | H | H | 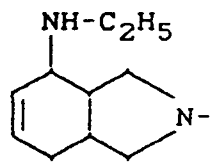 | CF |
| 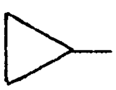 | H | H | 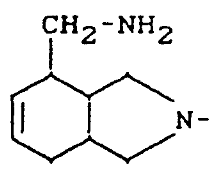 | CF |
| 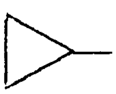 | H | H | 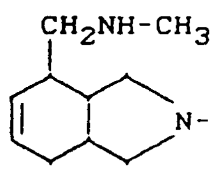 | CF |
| 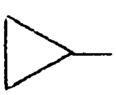 | H | H | 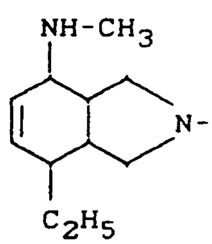 | CF |

| $R^1$ | $R^2$ | $X^2$ | Z | A |
|---|---|---|---|---|
| 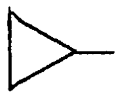 | H | H | 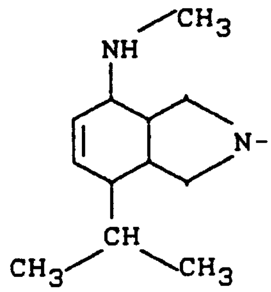 | CH |
| 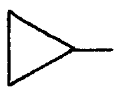 | H | H | 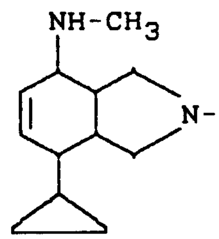 | CF |
| 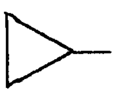 | H | $CH_3$ | 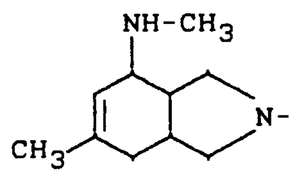 | CF |
| 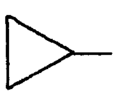 | H | H | 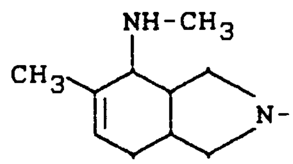 | CF |
| 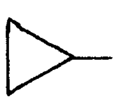 | H | H | 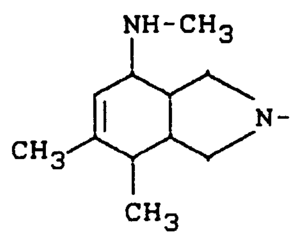 | CF |

| $R^1$ | $R^2$ | $X^2$ | Z | A |
|---|---|---|---|---|
| 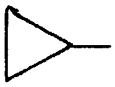 | H | H | 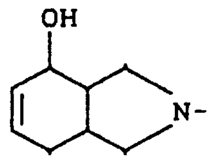 | CF |
| 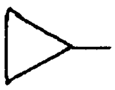 | H | H | 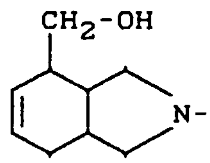 | N |
| 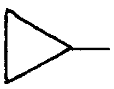 | H | H | 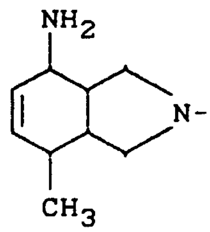 | N |
| 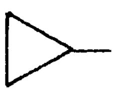 | H | H | 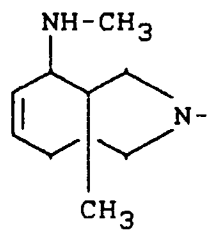 | CF |
| 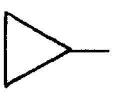 | $-C_2H_5$ | H | 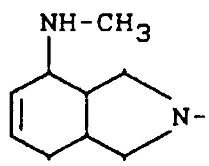 | CCl |
| 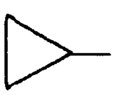 | $-CH_2CH_2-OH$ | H | 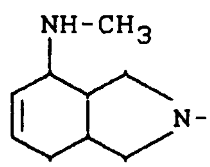 | CCl |

| $R^1$ | $R^2$ | $X^2$ | Z | A |
|---|---|---|---|---|
| 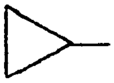 | $-CH_2CH_2-NH_2$ | H | 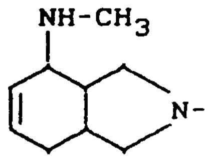 | CCl |
| 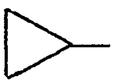 | $-CH_2CH_2-NH-CH_3$ | H | 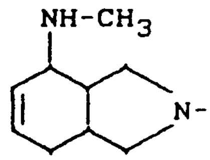 | CCl |
| 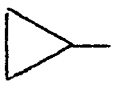 | $-CH_2CH_2N(CH_3)_2$ | H | 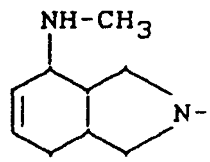 | CCl |
| $F-CH_2CH_2-$ | H | H | 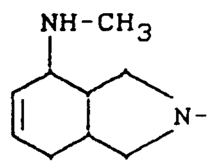 | CF |
| $CH_3-NH-$ | H | H | 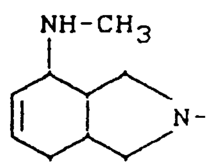 | CF |
| 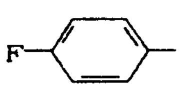 | H | H | 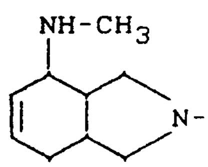 | CF |

Die erfindungsgemäßen Verbindungen wirken stark antibiotisch und zeigen bei geringer Toxizität ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und

resistente Keime aus. Bei ruhenden Bakterien, also Bakterien, die kein nachweisbares Wachstum zeigen, wirken die Verbindungen weit unterhalb von Konzentrationen bisher bekannter Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Pseudomonas aeruginosa, Enterococcus faecalis und Escherichia coli beobachtet werden.

Auch gegenüber Bakterien, die gegenüber vergleichbaren Substanzen als weniger empfindlich eingestuft werden, insbesondere resistenten Staphylococcus aureus, Escherichia coli, Pseudomonas aeruginosa und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt, Die beimpften Agarplatten wurden bei 37° C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich 7-(4-Amino-1,3-dihydro-isoindol-2-yl)-1-cyclopropyl-6,8-difluor-1,4-dihydro-4-oxo-3-chinolincarbonsäure (EP 343 560, Beispiel 2) aufgeführt.

Tabelle: MHK-Werte

| Teststamm: | Beispiel | 1 | 2 | 3 | 4 | 5 | 6 | 7 | 8 | 9B | 10 | 11 B | Ref*) |
|---|---|---|---|---|---|---|---|---|---|---|---|---|---|
| E.coli | Neumann<br>455/7 | 0,02<br>0,5 | 0,03<br>2 | 0,02<br>0,25 | 0,13<br>16 | 0,06<br>2 | 0,03<br>1 | 0,03<br>0,5 | 0,13<br>2 | 0,02<br>0,5 | 0,06<br>8 | 0,25<br>16 | 0,5<br>128 |
| Klebsiella sp. | 8085 | 0,06 | 0,13 | 0,03 | 0,25 | 0,06 | 0,06 | 0,06 | 0,25 | 0,03 | 0,13 | 0,5 | 4 |
| Morganella morg. | 932 | 0,06 | 0,13 | 0,03 | 0,5 | 0,06 | 0,06 | 0,03 | 0,25 | 0,06 | 0,25 | 0,5 | 1 |
| Providencia spp. | 12012 | 0,06 | 0,13 | 0,03 | 0,25 | 0,13 | 0,13 | 0,06 | 0,5 | 0,06 | 0,25 | 0,5 | 1 |
| Staphylococcus aureus | 1756 | 0,02 | 0,02 | 0,02 | 0,03 | 0,02 | 0,02 | 0,02 | 0,03 | 0,03 | 0,06 | 0,06 | 0,03 |
| | 133 | 0,02 | 0,02 | 0,02 | 0,03 | 0,02 | 0,02 | 0,02 | 0,03 | 0,03 | 0,06 | 0,06 | 0,03 |
| Enterococcus faecalis | 27101 | 0,03 | 0,06 | 0,02 | 0,13 | 0,03 | 0,06 | 0,02 | 0,13 | 0,03 | 0,25 | 0,25 | 0,5 |
| | 9790 | 0,03 | 0,06 | 0,02 | 0,13 | 0,03 | 0,06 | 0,02 | 0,13 | 0,02 | 0,25 | 0,25 | 1 |
| Pseudomonas aeruginosa | Walter | 1 | 2 | 0,5 | 16 | 2 | 2 | 1 | 4 | 1 | 4 | 8 | 128 |

*) Referenzverbindung:

7-(4-Amino-1,3-dihydro-isoindol-2-yl)-1-cyclopropyl-6,8-difluor-1,4-dihydro-4-oxo-3-chinolincarbonsäure (EP 343 560, Beispiel 2)

Herstellung der Zwischenprodukte:

Beispiel A

4-Methylamino-1,3,3a,4,7,7a-hexahydroisoindol

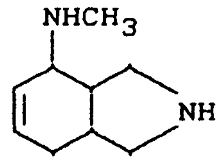

Methode I:

14,4 g (60 mmol) 70 %iges 1-(tert.-Butyloxycarbonylamino)-1,3-butadien [J.Org.Chem. 43, 2164 (1978)] werden als Lösung in 30 ml absolutem Tetrahydrofuran zu vorgelegten 10,1 g (60 mmol) N-Trimethylsilylmaleinimid [J.Org.Chem. 40, 24 (1975)] in 30 ml absolutem Tetrahydrofuran getropft. Nach dem Abklingen der exothermen Reaktion wird noch 1 Stunde unter Rückflußkühlung gekocht.

Die erkaltete Reaktionsmischung wird dann unter Stickstoff zu vorgelegten 7,6 g (0,2 mol) Lithiumaluminiumhydrid in 200 ml absolutem Tetrahydrofuran getropft. Dann wird 14 Stunden unter Rückflußkühlung gekocht. Zu der erkalteten Reaktionsmischung werden dann nacheinander 7,6 g Wasser in 23 ml Tetrahydrofuran, 7,6 g 10 %ige Natronlauge und 22,8 g Wasser getropft. Die Salze werden abfiltriert und das Filtrat im Vakuum eingeengt. Der Rückstand (10,3 g) wird bei 87° C/0,8 mbar destilliert.

Das Destillat wird in 80 ml absolutem Pentan aufgenommen, filtriert und das Produkt durch Abkühlen auf -70° C kristallisiert.

Ausbeute: 3,3 g, Schmelzpunkt: 72 - 82° C.

Durch Behandeln mit äquimolarer Menge 2n-Salzsäure erhält man 4-Methylamino-1,3,3a,4,7,7a-hexahydro-isoindol-Dihydrochlorid vom Schmelzpunkt 265-268° C (aus Methanol).

Methode II:

a) 4-(tert.-Butyloxycarbonylamino)-1,3-dioxo-1,3,3a,4,7,7a-hexahydroisoindol

48,0 g (0,5 mol) Maleinimid werden in 200 ml absolutem Tetrahydrofuran gelöst vorgelegt und 120 g (0,5 mol) ca. 70 %iges 1-(tert.-Butyloxycarbonylamino)-1,3-butadien als Lösung in 500 ml absolutem Tetrahydrofuran zugetropft, wobei die Temperatur bei 20 bis 30° C gehalten wird. Über Nacht wird bei Raumtemperatur nachgerührt. Dann wird eingeengt und aus Essigsäureethylester umkristallisiert. Es werden 57 g Produkt mit einem Schmelzpunkt von 177 bis 182° C erhalten. Aus der Mutterlauge werden weitere 13 g mit einem Schmelzpunkt von 158 bis 160° C erhalten.

b) 4-Methylamino-1,3,3a,4,7,7a-hexahydroisoindol

27,1 g (0,71 mol) Lithiumaluminiumhydrid werden unter Stickstoff in 300 ml absolutem Tetrahydrofuran vorgelegt und eine Lösung von 57 g (0,21 mol) 4-(tert.-Butyloxycarbonylamino)-1,3-dioxo-1,3,3a,4,7,7a-hexahydroisoindolin 570 ml absolutem Tetrahydrofuran zugetropft. Dann wird über Nacht unter Rückflußkühlung gekocht. Nach dem Erkalten werden nacheinander 27,1 g Wasser in 82 ml Tetrahydrofuran, 27,1 g 10 %ige Natronlauge und 81,3 g Wasser zu dem Ansatz getropft. Die Salze werden abgesaugt, mit Tetrahydrofuran gewaschen und das Filtrat im Vakuum eingeengt. Der Rückstand wird im Hochvakuum destilliert.

Ausbeute: 19,1 g

Beispiel B

4-Amino-1,3,3a,4,7,7a-hexahydro-isoindol

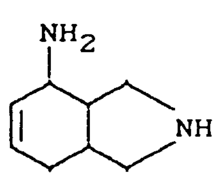

13,3 g (50 mmol) 4-tert.-Butyloxycarbonylamino-1,3-dioxo-1,3,3a,4,7,7a-hexahydro-isoindol (aus Beispiel A, Methode II) werden in 166 ml Trifluoressigsäure über Nacht bei Raumtemperatur gerührt. Dann wird die Trifluoressigsäure bei 10 mbar abdestilliert und der Rückstand bei 50° im Hochvakuum von Säureresten befreit. Anschließend wird in absolutem Tetrahydrofuran aufgenommen und im Vakuum eingeengt. Der Rückstand wird in 100 ml absolutem Tetrahydrofuran aufgenommen und unter Stickstoff zu einer Lösung von 11,3 g (0,3 mol) Lithiumaluminiumhydrid in 300 ml absolutem Tetrahydrofuran getropft. Dann wird 16 Stunden unter Rückflußkühlung gekocht. Nach dem Erkalten werden nacheinander 11,3 g Wasser in 34 ml Tetrahydrofuran, 11,3 ml 10 %ige Natronlauge und 34 ml Wasser zugetropft. Der Niederschlag wird abgesaugt und mit Tetrahydrofuran gewaschen. Das Filtrat wird eingeengt und der Rückstand destilliert.
Ausbeute: 2,2 g, Gehalt: 92 % (gaschromatographisch bestimmt)
Siedepunkt: 70° /0,2 mbar

Beispiel C

7-Methyl-4-methylamino-1,3,3a,4,7,7a-hexahydro-isoindol

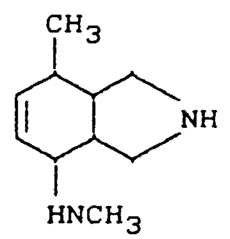

In Analogie zu Beispiel A, Methode I werden 21,9 g (0,12 mol) 1-(tert.-Butyloxycarbonylamino)-1,3-pentadien mit 20,3 g (0,12 mol) N-Trimethylsilyl-maleinimid umgesetzt und anschließend mit 15,2 g (0,4 mol) Lithiumaluminiumhydrid reduziert. Das Rohprodukt wird aus Tetrahydrofuran umkristallisiert.
Ausbeute: 6,2 g, Schmelzpunkt: 106 - 108° C.

Beispiel D

5-Brom-1-cyclopropyl-6,7,8-trifluor-1,4-dihydro-4-oxo-3-chinolincarbonsäure

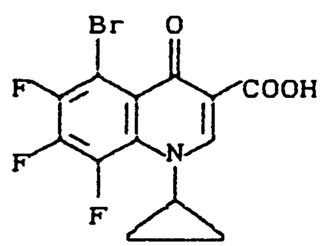

a) 2-Brom-3,4,5,6-tetrafluor-benzoylchlorid
365 g (1,33 mol) 2-Brom-3,4,5,6-tetrafluorbenzoesäure [Tetrahedron 23, 4719 (1967)] werden in 2 l Thionylchlorid eingetragen und die Mischung 11 Stunden bis zum Aufhören der Gasentwicklung unter Rückfluß erhitzt. Überschüssiges Thionylchlorid wird im Vakuum abgezogen und der Rückstand destilliert.
Ausbeute: 330 g (85 % der Theorie)
Siedepunkt: 81 - 85° C/3-5 mbar
b) (2-Brom-3,4,5,6-tetrafluor-benzoyl)-malonsäurediethylester
Man legt 15,9 g (0,167 mol) Magnesiumchlorid in 150 ml wasserfreiem Acetonitril (über Zeolith getrocknet) vor und läßt unter Kühlung 26,9 g (0,167 mol) Malonsäurediethylester zutropfen. Man kühlt auf 0° C, tropft 46 ml (33,7 g = 0,33 mol) Triethylamin zu und rührt 30 Minuten nach. Anschließend werden 48,9 g (0,168 mol) 2-Brom-3,4,5,6-tetrafluorbenzoylchlorid zugetropft, noch 1 Stunde bei 0° C

nachgerührt und die Mischung über Nacht auf Raumtemperatur gebracht. Man versetzt mit 100 ml 5n-Salzsäure, extrahiert dreimal mit Methylenchlorid, trocknet mit $Na_2SO_4$ und engt im Vakuum ein.
Rohausbeute: 62,7 g
c) (2-Brom-3,4,5,6-tetrafluor-benzoyl)-essigsäureethylester
60 g rohen (2-Brom-3,4,5,6-tetrafluor-benzoyl)-malonsäurediethylester trägt man in 150 ml Wasser ein, versetzt mit 0,6 g 4-Toluolsulfonsäure und erhitzt 6 Stunden unter Rückfluß. Man extrahiert mit Methylenchlorid, wäscht mit Wasser, trocknet mit $Na_2SO_4$ und engt ein.
Rohausbeute: 46 g
Siedepunkt (Probedestillation im Kugelrohr): 150 - 160° C (Ofen)/3 mbar;
Massenspektrum: $^m/e$ 342 ($M^+$), 297 ($M^+$-$OC_2H_5$), 263 ($M^+$-Br), 257, 255 ($M^+$-$CH_2CO_2C_2H_5$), 235 (263-28).
d) 2-(2-Brom-3,4,5,6-tetrafluor-benzoyl)-3-ethoxyacrylsäureethylester
Man trägt 45 g rohen (2-Brom-3,4,5,6-tetrafluor-benzoyl)-essigsäureethylester in 32,2 g (0,31 mol) Essigsäureanhydrid und 28,4 g (0,19 mol) Orthoameisensäuretriethylester ein und erhitzt 2 Stunden unter Rückfluß. Überschüssiges Reagens wird zunächst im Vakuum, anschließend im Hochvakuum (Bad bis 120 - 130° C) abgezogen und das Rohprodukt zur nächsten Stufe umgesetzt.
Rohausbeute: 50,7 g
e) 2-(2-Brom-3,4,5,6-tetrafluor-benzoyl)-3-cyclopropylamino-acrylsäureethylester
50,7 g rohes Produkt aus Stufe d) werden in 90 ml Ethanol unter Eiskühlung mit 8,6 g (0,15 mol) Cyclopropylamin tropfenweise versetzt, die Mischung bei Raumtemperatur nachgerührt, über Nacht stehengelassen, nochmal gut gekühlt, das Kristallisat abgesaugt, mit kaltem Ethanol gewaschen und getrocknet.
Ausbeute: 29 g (42 % über 4 Stufen)
Schmelzpunkt: 103 - 105° C (aus Ethanol)
f) 5-Brom-1-cyclopropyl-6,7,8-trifluor-1,4-dihydro-4-oxo-3-chinolincarbonsäureethylester
28 g (68 mmol) 2-(2-Brom-3,4,5,6-tetrafluor-benzoyl)-3-cyclopropylamino-acrylsäureethylester werden in 88 ml DMF mit 6,9 g (164 mmol) Natriumfluorid 6 Stunden unter Rückfluß erhitzt. Die Mischung wird nach dem Abkühlen in Wasser eingegossen, der ausgefallene Niederschlag (rot) abgesaugt, mit viel Wasser gewaschen und bei 80° C im Umluftschrank getrocknet.
Rohausbeute: 27,3 g
Schmelzpunkt:150 - 175° C; nach Umkristallisation aus Glykolmonomethylether: Schmelzpunkt: 187 - 191° C
g) 5-Brom-1-cyclopropyl-6,7,8-trifluor-1,4-dihydro-4-oxo-3-chinolincarbonsäure
26,7 g (68 mmol) roher 5-Brom-1-cyclopropyl-6,7,8-trifluor-1,4-dihydro-4-oxo-3-chinolincarbonsäureethylester werden in eine Mischung aus 165 ml Essigsäure, 110 ml Wasser und 18 ml konzentrierter Schwefelsäure eingetragen und 2 Stunden unter Rückfluß erhitzt. Das abgekühlte Reaktionsgemisch wird auf Eiswasser gegossen, der ausgefallene Niederschlag abgesaugt, mit viel Wasser nachgewaschen und im Umluftschrank bei 80° C getrocknet.
Ausbeute: 19,7 g (80 % der Theorie)
Schmelzpunkt: 208 - 210° C (unter Zersetzung);
nach Umkristallisation aus Glykolmonomethylether:
Schmelzpunkt: 212 - 214° C (unter Zersetzung)
$^1$H-NMR (DMSO): 8,73 s (1H an C-2), 4,16 m (1H, Cyclopropyl), 1,2 m (4H, Cyclopropyl) [ppm].
Massenspektrum: $^m/e$ 361 ($M^+$), 343 ($M^+$-$H_2O$), 317 (M-$CO_2$), 41 (100 %, $C_3H_5$).

## Beispiel E

5-Brom-1-(2,4-difluorphenyl)-6,7,8-trifluor-1,4-dihydro-4-oxo-3-chinolincarbonsäure

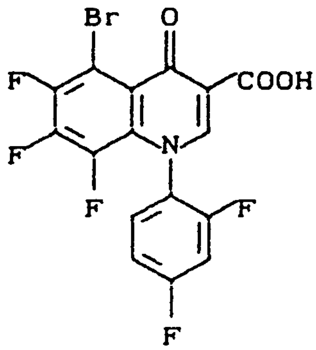

Analog Beispiel De, Df und Dg erhält man mit 2,4-Difluoranilin:

a) 2-(2-Brom-2,3,4,5-tetrafluor-benzoyl)-3-(2,4-difluorphenylamino)-acrylsäureethylester, Schmelzpunkt: 116 - 117° C,

b) 5-Brom-1-(2,4-difluorphenyl)-6,7,8-trifluor-1,4-dihydro-4-oxo-3-chinolincarbonsäure-ethylester, Schmelzpunkt: 190 - 192° C (unter Zersetzung)

c) 5-Brom-1-(2,4-difluorphenyl)-6,7,8-trifluor-1,4-dihydro-4-oxo-3-chinolincarbonsäure, Schmelzpunkt: 237 - 240° C (unter Zersetzung),
Massenspektrum: $^{m}/e$ 433 ($M^{+}$), 389 (100 %, $M^{+}$-$CO_2$).

Beispiel F

5,8-Dichlor-1-cyclopropyl-6,7-difluor-1,4-dihydro-4-oxo-3-chinolincarbonsäure

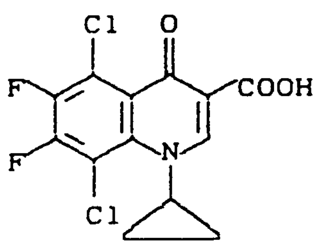

a) 3-Chlor-2,4,5-trifluor-6-nitrobenzoesäure
210,5 g (1 mol) 3-Chlor-2,4,5-trifluorbenzoesäure (DE 3 420 796) werden in 1 000 ml konzentrierter Schwefelsäure vorgelegt und auf 40° C erwärmt. Dazu tropft man 189 g (3 mol) wasserfreie Salpetersäure, wobei die Innentemperatur zwischen 35 und 45° C gehalten wird. Nach vollständiger Zugabe wird noch weitere 24 Stunden bei Raumtemperatur gerührt. Die Suspension wird auf 2 kg Eis gegeben. Der ausgefallene Feststoff wird abgesaugt und mit Wasser gewaschen und über KOH im Exsikkator getrocknet.
Ausbeute: 157,5 g (62 % der Theorie)
Schmelzpunkt: 160 - 162° C (aus Chlorbenzol)

b) 2-Amino-5-chlor-3,4,6-trifluorbenzoesäure
63,9 g (0,25 mol) 3-Chlor-2,4,5-trifluor-6-nitrobenzoesäure und 15 g Raney-Nickel werden in 450 ml Ethanol suspendiert. Bei 10 - 15° C und einem Wasserstoffdruck von 5 - 10 bar wird bis zum Ende der Wasserstoffaufnahme hydriert. Die rohe Reaktionsmischung wird auf 1 l Wasser gegeben. Durch Zugabe von 10 %iger Natronlauge wird die ausgefallene Carbonsäure in Lösung gebracht. Nach Absaugen des Katalysators wird durch Zugabe von halbkonzentrierter Salzsäure ein pH-Wert von 2 - 3 eingestellt. Der Niederschlag wird abgesaugt, mit Wasser gewaschen und über KOH im Exsikkator getrocknet.
Ausbeute: 51,8 g (92 % der Theorie)
Schmelzpunkt: 183 - 185° C

c) 2,5-Dichlor-3,4,6-trifluorbenzoesäure

45,1 g (0,2 mol) 2-Amino-5-chlor-3,4,6-trifluorbenzoesäure werden in 300 ml wasserfreier Essigsäure vorgelegt. Bei einer Innentemperatur von 25 - 30° C werden 27,9 g (0,22 mol) Nitrosylschwefelsäure in 150 ml konzentrierter Schwefelsäure gelöst zugetropft. Anschließend wird 1 Stunde bei Raumtemperatur gerührt. Zur Entfernung des Nitritüberschusses wird bis zum Ende der Stickstoffentwicklung eine bei Raumtemperatur gesättigte Lösung von Amidosulfonsäure zugetropft.

10 g (0,1 mol) Kupfer(I)chlorid werden in 300 ml halbkonzentrierter Salzsäure vorgelegt. Dazu tropft man bei einer Innentemperatur von 10 - 15° C die wie voranstehend beschrieben hergestellte Lösung des Diazoniumsalzes. Anschließend wird noch 2 Stunden bei Raumtemperatur gerührt. Die ausgefallenen Kristalle werden mit Wasser gewaschen und über KOH im Exsikkator getrocknet.

Ausbeute: 26,8 g (55 % der Theorie)

Schmelzpunkt: 119 - 120° C

d) 2,5-Dichlor-3,4,6-trifluorbenzoylchlorid

160 ml Thionylchlorid werden bei Raumtemperatur vorgelegt. Dazu werden unter Rühren 36,8 g (0,15 mol) 2,5-Dichlor-3,4,6-trifluorbenzoesäure portionsweise zugegeben. Nach Zugabe von 0,5 ml DMF wird langsam auf 80 - 90° C erhitzt und 5 Stunden bei dieser Temperatur gerührt. Anschließend wird eingeengt und im Hochvakuum destilliert.

Ausbeute: 31,6 g (80 % der Theorie)

Siedepunkt: 65° C/1,4 mbar

e) (2,5-Dichlor-3,4,6-trifluorbenzoyl)-essigsäureethylester

30,6 g (0,15 mol) Malonsäure-ethyl-trimethylsilylester werden in 100 ml Diethylether vorgelegt. Bei -78° C werden 60 ml einer 2,5 m Lösung von n-Butyllithium (0,15 mol) zugetropft. Nach vollständiger Zugabe wird noch 10 Minuten bei dieser Temperatur gerührt. Anschließend tropft man 26,4 g (0,1 mol) 2,5-Dichlor-3,4,6-trifluorbenzoylchlorid in 100 ml Dimethoxyethan gelöst zu.

Nach vollständiger Zugabe läßt man auf Raumtemperatur kommen und rührt 20 Stunden bei dieser Temperatur. Anschließend werden 100 ml Wasser zugetropft, die organische Phase wird abgetrennt, mit Natriumsulfat getrocknet und eingeengt. Der Rückstand wird in 200 ml Petrolether aufgenommen und abgesaugt. Der abgesaugte Feststoff wird verworfen. Das Filtrat wird eingeengt und im Hochvakuum destilliert.

Ausbeute: 21,3 g (68 % der Theorie)

Siedepunkt: 115 - 120° C/0,8 mbar

f) 2-(2,5-Dichlor-3,4,6-trifluorbenzoyl)-3-ethoxyacrylsäureethylester

Eine Mischung aus 31,5 g (0,1 mol) (2,5-Dichlor-3,4,6-trifluorbenzoyl)-essigsäureethylester, 22,2 g (0,15 mol) Orthoameisensäuretriethylester und 25,5 g (0,25 mol) Essigsäureanhydrid wird 2 Stunden zum Rückfluß erhitzt (150° C Heizbadtemperatur). Leichtsiedende Bestandteile werden zunächst im Wasserstrahlvakuum, anschließend im Hochvakuum bis zu einer Sumpftemperatur von 130° C abdestilliert. Der Rückstand wird als Rohprodukt weiterverwendet.

Ausbeute: 34,7 g (94 % der Theorie)

g) 2-(2,5-Dichlor-3,4,6-trifluorbenzoyl)-3-cyclopropylaminoacrylsäureethylester

37,1 g (0,1 mol) 2-(2,5-Dichlor-3,4,6-trifluorbenzoyl)-3-ethoxy-acrylsäureethylester werden in 100 ml Ethanol vorgelegt. Dazu tropft man 6,8 g (0,12 mol) Cyclopropylamin in 10 ml Ethanol gelöst zu. Anschließend wird 4 Stunden bei Raumtemperatur gerührt. Der Niederschlag wird abgesaugt, mit Petrolether gewaschen und an der Luft getrocknet.

Ausbeute: 30,6 g (80 % der Theorie)

Schmelzpunkt: 113 - 115° C

h) 5,8-Dichlor-1-cyclopropyl-6,7-difluor-1,4-dihydro-4-oxo-3-chinolincarbonsäureethylester

19,1 g (0,05 mol) 2-(2,5-Dichlor-3,4,6-trifluorbenzoyl)-3-cyclopropylamino-acrylsäureethylester werden zusammen mit 4,2 g (0,1 mol) Natriumfluorid in 100 ml N-Methyl-2-pyrrolidon 5 Stunden auf 160 - 170° C erhitzt. Nach Abkühlung wird der Ansatz auf 200 ml Wasser gegeben. Der ausgefallene Feststoff wird abgesaugt, mit Wasser gewaschen und bei 80° C im Trockenschrank getrocknet.

Ausbeute: 17,0 g (94 % der Theorie)

Schmelzpunkt: 185 - 186° C

i) 5,8-Dichlor-1-cyclopropyl-6,7-difluor-1,4-dihydro-4-oxo-3-chinolincarbonsäure

18,1 g (0,05 mol) 5,8-Dichlor-1-cyclopropyl-6,7-difluor-1,4-dihydro-4-oxo-3-chinolincarbonsäureethylester werden in einer Mischung aus 360 ml Essigsäure, 300 ml Wasser und 40 ml konzentrierter Schwefelsäure 2,5 Stunden bei 80° C gerührt. Nach Abkühlung werden die ausgefallenen Kristalle abgesaugt, mit Wasser gewaschen und bei 80° C im Trockenschrank getrocknet.

Ausbeute: 15,7 g (94 % der Theorie)

Schmelzpunkt: 219 - 220° C

$^1H$-NMR ($CDCl_3$): 8,91 (s, 1H, C2), 4,35 (m, 1H, Cyclopropyl-CH), 1,35 und 1,0 ppm (2m, je 2H, Cyclopropyl-$CH_2$).

Beispiel G

5,8-Dichlor-1-(2,4-difluorphenyl)-6,7-difluor-1,4-dihydro-4-oxo-3-chinolincarbonsäure

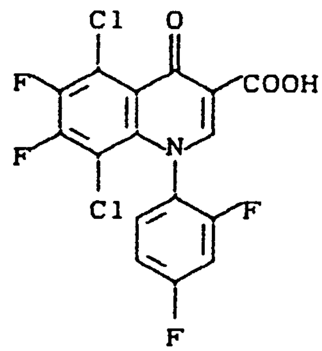

Analog Beispiel Fg, Fh und Fi erhält man mit 2,4-Difluoranilin:
a) 2-(2,5-Dichlor-3,4,6-trifluorbenzoyl)-3-(2,4-difluorphenylamino)-acrylsäureethylester
Schmelzpunkt: 103 - 104° C
b) 5,8-Dichlor-1-(2,4-difluorphenyl)-6,7-difluor-1,4-dihydro-4-oxo-3-chinolincarbonsäureethylester
Schmelzpunkt: 220 - 221° C
c) 5,8-Dichlor-1-(2,4-difluorphenyl)-6,7-difluor-1,4-dihydro-4-oxo-3-chinolincarbonsäure
Schmelzpunkt: 223 - 224° C

Beispiel H

7-Isopropyl-4-methylamino-1,3,3a,4,7,7a-hexahydroisoindol

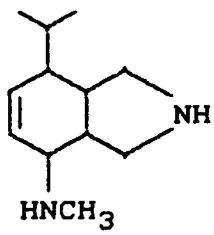

50 g (0,24 mol) 1-(tert.-Butyloxycarbonylamino)-5-methyl-1,3-hexadien werden zusammen mit 23 g (0,24 mol) Maleinimid in 75 ml Ethanol und 75 ml Wasser 24 Stunden unter Rückfluß gerührt. Nach dem Abkühlen saugt man den Feststoff ab, wäscht mit Wasser nach und erhält nach dem Trocknen 56,3 g (76 % der Theorie) eines Feststoffes mit dem Schmelzpunkt 192-195° C. 15 g (0,049 mol) werden zusammen mit 11 g (0,29 mol) Lithiumaluminiumhydrid in 300 ml Tetrahydrofuran 10 Stunden unter Rückfluß gerührt. Nach dem Abkühlen wird mit 10 ml Wasser, 10 ml 10 proz. Natronlauge und zuletzt 30 ml Wasser hydrolysiert. Man saugt von Niederschlag ab, wäscht mit Tetrahydrofuran nach, und engt die vereinigten Filtrate zur Trockne ein. Es werden 8,7 g eines Feststoffes erhalten, der durch Kristallisation (Petrolether-Essigsäureethylester = 1:5) gereinigt wird.
Ausbeute: 43,5 g (51 % der Theorie),
Schmelzpunkt: 76-81° C.

Beispiel I

4-Amino-7-isopropyl-1,3,3a,4,7,7a-hexahydro-isoindol

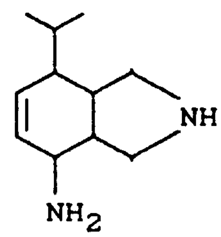

Die Darstellung erfolgt analog Beispiel B.
$^1$H-NMR (200 MHz, $CDCl_3$): δ = 0,95 (6H); 2,3-2,7 (m, 7H); 5,75 (2H).
MS: m/e (% rel. Int.): 180 [$M^+$](7); 163 (45); 120 (100); 67 (100).

Beispiel J

4-Hydroxymethyl-1,3,3a,4,7,7a-hexahydro-isoindol

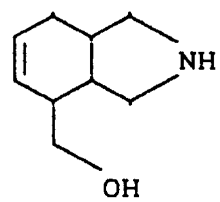

25 g (0,22 mol) 2,4-Pentadiencarbonsäuremethylester werden in 100 ml Dioxan mit 20 g (0,21 mol) Maleinsäureimid 40 Stunden unter Rückfluß gerührt. Das nach dem Einengen erhaltene Öl (51 g) wird in 350 ml Tetrahydrofuran mit 20 g (0,52 mol) Lithiumaluminiumhydrid 16 Stunden unter Rückfluß gerührt. Nach dem Abkühlen wird mit 63 ml Wasser, 63 ml 10 proz. Natronlauge und zuletzt 60 ml Wasser hydrolysiert, vom Niederschlag abgesaugt und dieser noch mehrmals mit Tetrahydrofuran nachgewaschen. Die vereinigten Filtrate werden eingeengt und im Hochvakuum destilliert.
Ausbeute: 10 g (30 % der Theorie)
Siedepunkt: 96-115° C / 0,07 mbar.

Beispiel K

4-Methylaminomethyl-1,3,3a,4,7,7a-hexahydroisoindol

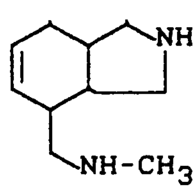

a) 1-tert.-Butyloxycarbonylamino-2,4-pentadien
Durch Umsetzung von 1-Amino-2,4-pentadien (P.A. Grieco et al,, Tetrahedron 42, 2847 [1986]) mit Ditert.-butylcarbonat in Dioxan bei Raumtemperatur während 12 Stunden bei pH 8-10 erhält man 1-tert.-Butyloxycarbonylamino-2,4-pentadien als helles Öl in quantitativer Ausbeute.
$^1$H-NMR (200 MHz, $CDCl_3$): δ = 1,45 (9H), 3,78 (2H); 4,65 (br., 1H); 5,05-5,21 (m, 2H); 5.60-5,75 (m, 1H); 6.08-6.42 ppm (m, 2H).

b) 4-tert.-Butyloxycarbonylaminomethyl-1,3-dioxo-1,3,3a,4,7,7a-hexahydro-isoindol
30 g (0,16 mol) 1-tert.-Butyloxycarbonylamino-2,4-pentadien werden zusammen mit 16 g (0,16 mol) Maleinimid in 120 ml Dioxan 12 Stunden bei Rückfluß gerührt. Nach dem Abkühlen engt man zur Hälfte ein und saugt den Feststoff ab.
Ausbeute: 35,3 g (76 %)
Schmelzpunkt: 197,5-198,5° C.
c) 4-Methylaminomethyl-1,3,3a,4,7,7a-hexahydroisoindol
Man reduziert 4-tert.-Butyloxycarbonylaminomethyl-1,3-dioxo-1,3,3a,4,7,7a-hexahydroisoindol mit Lithiumaluminiumhydrid analog wie in Beispiel A, Methode IIb beschrieben: gelbes Öl.
Siedepunkt = 78° C/0,05 mbar.

Beispiel L

4-Aminomethyl-1,3,3a,4,7,7a-hexahydro-isoindol

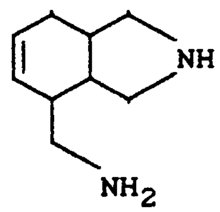

Man setzt 4-tert.-Butyloxycarbonylaminomethyl-1,3-dioxo-1,3,3a,4,7,7a-hexahydroisoindol analog wie in Beispiel B beschrieben ein.
Siedepunkt = 135-140° C/0,1 mbar.

Beispiel M

6-Methyl-4-methylamino-1,3,3a,4,7,7a-hexahydro-isoindol

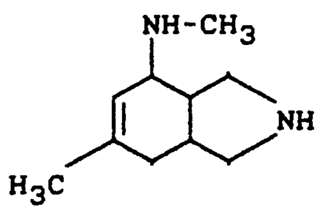

a) 4-(tert.-Butyloxycarbonylamino)-1,3-dioxo-6-methyl-1,3,3a,4,7,7a-hexahydro-iso-indol
Entsprechend Beispiel A/Methode IIa setzt man mit 1-tert.-Butyloxycarbonylamino-3-methyl-1,3-butadien in Dioxan um.
Schmelzpunkt: 135° C
b) 6-Methyl-4-methylamino-1,3,3a,4,7,7a-hexahydro-isoindol
In Analogie zu Beispiel B werden 5,6 g (20 mmol) des Produkts aus Beispiel Ma) mit 2,2 g (60 mmol) Lithiumaluminiumhydrid in 60 ml Tetrahydrofuran 15 Stunden unter Rückfluß erhitzt. Bei der destillativen Aufarbeitung werden 1,2 g des Produkts vom Siedepunkt 68-71° C/0,2-0,3 mbar erhalten.

Beispiel N

4-Amino-7-methyl-1,3,3a,4,7,7a-hexahydro-isoindol

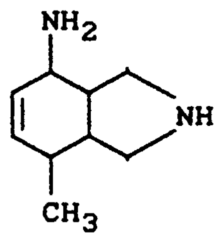

a) 4-(tert.-Butyloxycarbonylamino)-1,3-dioxo-7-methyl-1,3,3a,4,7,7a-hexahydro-isoindol
Entsprechend Beispiel A/Methode IIa setzt man mit 1-tert.-Butyloxycarbonylamino-1,3-pentadien ein und kristallisiert das Reaktionsprodukt aus Dioxan um.
Ausbeute: 79 %
Schmelzpunkt: 208-211° C
b) 4-Amino-7-methyl-1,3,3a,4,7,7a-hexahydro-isoindol
Entsprechend Beispiel B setzt man das Produkt aus Beispiel Na) ein und erhält das freie Amin als Öl vom Siedepunkt 83-92° C/0,1 mbar, das beim Stehen kristallisiert.
Gehalt: 90 %ig (nach Gaschromatogramm)

Beispiel O

4-Amino-7-cyclopropyl-1,3,3a,4,7,7a-hexahydro-isoindol

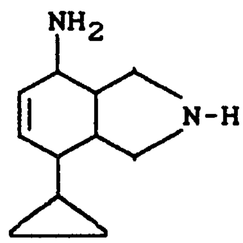

a) 4-(tert.-Butoxycarbonylamino)-7-cyclopropyl-1,3-dioxo-1,3,3a,4,7,7a-hexahydro-isoindol
1-tert.-Butoxycarbonylamino-4-cyclopropyl-1,3-butadien (hergestellt analog der in J Org. Chem. 43, 2164 [1978] beschriebenen Methode; IR ($CCl_4$): 330, 1720, 1605 $cm^{-1}$) wird analog Beispiel A/Methoce II umgesetzt.
Schmelzpunkt: 195,5-196,5° C.
b) 4-Amino-7-cyclopropyl-1,3,3a,4,7,7a-hexyhydro-isoindol
Das Produkt aus Beispiel Oa wird analog Beispiel B mit Lithiumaluminiumhydrid zu einem viscosen Öl umgesetzt.
FAB-MS (Glycerin/DMSO): m/e 179 ($[M+H]^+$).

Herstellung der Wirkstoffe:

Beispiel 1

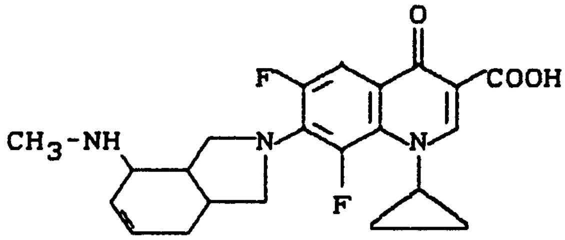

Eine Mischung von 1.42 g (5 mmol) 1-Cyclopropyl-6,7,8-trifluor-1,4-dihydro-4-oxo-3-chinolincarbonsäure in 10 ml Acetonitril und 5 ml Dimethylformamid mit 560 mg (5 mmol) 1,4-Diazabicyclo[2.2.2]octan, 0,84 g (5,5 mmol) 4-Methylamino-1,3,3a,4,7,7a-hexahydro-isoindol wird 1 Stunde unter Rückfluß erhitzt. Die Mischung wird eingeengt, der Rückstand mit 60 ml Wasser verrührt (pH 6-7), der ausgefallene Niederschlag abgesaugt, mit Wasser gewaschen und bei 90° C im Hochvakuum getrocknet.
Ausbeute: 1,61 g (86,3 % der Theorie) 1-Cyclopropyl-6,8-difluor-1,4-dihydro-7-(4-methylamino-1,3,3a,4,7,7a-hexahydro-isoindol-2-yl)-4-oxo-3-chinolincarbonsäure,
Schmelzpunkt: 233 - 235° C (unter Zersetzung) (aus Glykolmonomethylether).

Analog Beispiel 1 werden hergestellt:

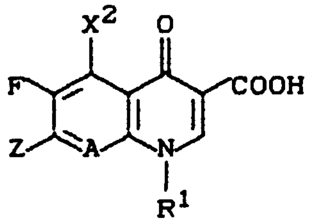

| Bei-spiel | Z | $R^1$ | A | $X^2$ | Schmelzpunkt (unter Zersetzung) [°C] |
|---|---|---|---|---|---|
| 2 | NH-$CH_3$ | | CH | H | 216-219 (aus GME) |
| 3 | 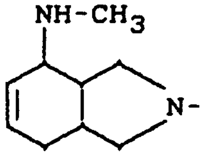 | 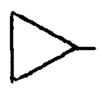 | CCl | H | 199-201 |
| 4 | | | CH | $CH_3$ | 198-200 (aus GME) |
| 5 | NH-$CH_3$ | | CH | H | 211-214 |
| 6 | 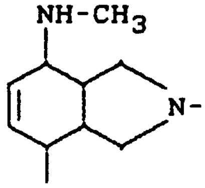 | 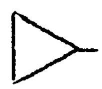 | CF | H | 213-217 (aus DMF) |
| 7 | | | CCl | H | 139-143 (aus GME) |
| 8 | $CH_3$ rac. | $C_2H_5$- | CF | H | 222-224 (aus GME) |

Beispiel 9

A. 1,5 g (5 mmol) 8-Chlor-1-cyclopropyl-6,7-difluor-1,4-dihydro-4-oxo-3-chinolincarbonsäure werden in einer Mischung aus 15 ml Acetonitril und 7,5 ml Dimethylformamid mit 1,2 g (5,3 mmol) 4-Methylamino-1,3,3a,4,7,7a-hexahydro-isoindol-Dihydrochlorid und 1,65 g (15 mmol) 1,4-Diazabicyclo[2.2.2]octan 1 Stunde unter Rückfluß erhitzt. Die Suspension wird eingeengt, der Rückstand mit Wasser verrührt, der ungeloste Rückstand abgesaugt, mit Wasser gewaschen und bei 100° C getrocknet.
Ausbeute: 1,98 g (92 % der Theorie) 8-Chlor-1-cyclopropyl-6-fluor-1,4-dihydro-7-(4-methylamino-1,3,3a,4,7,7a-hexahydro-isoindol-2-yl)-4-oxo-3-chinolincarbonsäure,
Schmelzpunkt: ab 198° C (unter Zersetzung); identisch mit dem Produkt aus Beispiel 3.

B. 8,0 g (18,5 mmol) des Produktes aus Stufe A werden in 50 ml Wasser suspendiert, mit 20 ml 1n-Salzsäure versetzt und das erhaltene Hydrochlorid isoliert und bei 80° C im Vakuum über Kaliumhydroxid getrocknet. Das Rohprodukt (5,9 g) wird aus Glykolmonomethylether umkristallisiert.
Ausbeute: 4,4 g (51 % der Theorie) 8-Chlor-1-cyclopropyl-6-fluor-1,4-dihydro-7-(4-methylamino-1,3,3a,4,7,7a-hexahydro-isoindol-2-yl)-4-oxo-3-chinolincarbonsäure-Hydrochlorid
Schmelzpunkt: 248 - 253° C (unter Zersetzung)

Beispiel 10

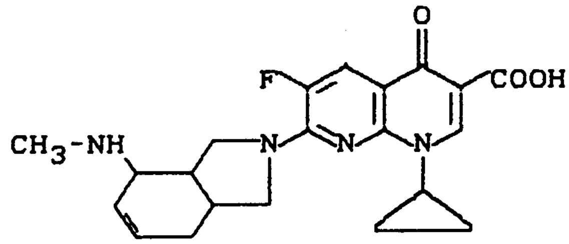

x HCl

282 mg (1 mmol) 7-Chlor-1-cyclopropyl-6-fluor-1,4-dihydro-4-oxo-1,8-naphthyridin-3-carbonsäure werden in 3 ml Acetonitril mit 310 mg (2 mmol) 4-Methylamino-1,3,3a,4,7,7a-hexahydro-isoindol 1 Stunde bei Raumtemperatur gerührt. Der ungelöste Feststoff wird abgesaugt, mit Wasser und Acetonitril gewaschen und bei 120° C im Hochvakuum getrocknet. Man erhält 0,4 g 1-Cyclopropyl-6-fluor-1,4-dihydro-7-(4-methylamino-1,3,3a,4,7,7a-hexahydro-isoindol-2-yl)-4-oxo-1,8-naphthyridin-3-carbonsäure als Rohprodukt, das in 5 ml halbkonzentrierter Salzsäure in der Wärme gelöst wird. Das Hydrochlorid wird durch Zugabe von Ethanol ausgefällt, abgesaugt und bei 120° C im Hochvakuum getrocknet.
Ausbeute: 190 mg (44 % der Theorie) 1-Cyclopropyl-6-fluor-1,4-dihydro-7-(4-methylamino-1,3,3a,4,7,7a-hexahydro-isoindol-2-yl)-4-oxo-1,8-naphthyridin-3-carbonsäure-Hydrochlorid
Schmelzpunkt: 300 - 305° C (unter Zersetzung)

Beispiel 11

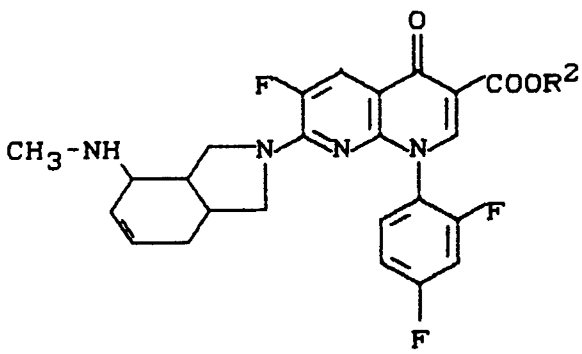

A. $R^2 = C_2H_5$

B. $R^2$ = H x HCl

A. Methode I:
1,9 g (5 mmol) 7-Chlor-1-(2,4-difluorphenyl)-6-fluor-1,4-dihydro-4-oxo-1,8-naphthyridin-3-carbonsäureethylester werden in 20 ml Acetonitril mit 560 mg (5 mmol) 1,4-Diazabicyclo[2.2.2]octan und 890 mg (5,3 mmol) 90 %igem 4-Methylamino-1,3,3a,4,7,7a-hexahydro-isoindol versetzt. Die Mischung wird 3 Stunden bei Raumtemperatur gerührt, anschließend im Vakuum eingeengt und der Rückstand mit 80 ml Wasser verrührt. Der ungelöste Rückstand wird abgesaugt, mit Wasser gewaschen und getrocknet.
Ausbeute: 1,6 g (64 % der Theorie) 1-(2,4-Difluorphenyl)-6-fluor-1,4-dihydro-7-(4-methylamino-1,3,3a,4,7,7a-hexahydro-isoindol-2-yl)-4-oxo-1,8-naphthyridin-3-carbonsäureethylester,
Schmelzpunkt: 173 - 176° C (unter Zersetzung)
Methode II:
382 mg (1 mmol) 7-Chlor-1-(2,4-difluorphenyl)-6-fluor-1,4-dihydro-4-oxo-1,8-naphthyridin-3-carbonsäureethylester werden in 1 ml Acetonitril und 1,5 ml Dimethylformamid mit 460 mg (3 mmol) 1,8-Diazabicyclo[5.4.0]undec-7-en (DBU) und 240 mg (1,1 mmol) 4-Methylamino-1,3,3a,4,7,7a-hexahydroisoindol-Dihydrochlorid versetzt und 1 Stunde bei Raumtemperatur gerührt. Man engt im Vakuum ein, versetzt mit 10 ml Wasser und saugt den ausgefallenen Niederschlag ab.
Ausbeute: 460 mg (92 % der Theorie) des Esters vom Schmelzpunkt 175-178° C (unter Zersetzung); identisch mit dem nach Methode I hergestellten Produkt.
Massenspektrum: $^{m}/e$ 498 ($M^{+}$), 467 ($M^{+}$-31), 416, 395, 370, 28.
B. 0,47 g (0,9 mmol) des Produktes aus Stufe A werden in einer Mischung aus 4,7 ml Essigsäure und 3,8 ml halbkonzentrierter Salzsäure 3 Stunden unter Rückfluß erhitzt. Die Mischung wird bis zur Trockne

eingeengt, der Rückstand mit wenig Ethanol verrührt, der ungelöste Niederschlag abgesaugt, mit Ethanol gewaschen und bei 80° C im Hochvakuum getrocknet.

Ausbeute: 0,30 g (63 % der Theorie) 1-(2,4-Difluorphenyl)-6-fluor-1,4-dihydro-7-(4-methylamino-1,3,3a,4,7,7a-hexahydro-isoindol-2-yl)-4-oxo-1,8-naphthyridin-3-carbonsäure-Hydrochlorid

Schmelzpunkt: 286 - 287° C (unter Zersetzung).

Beispiel 12

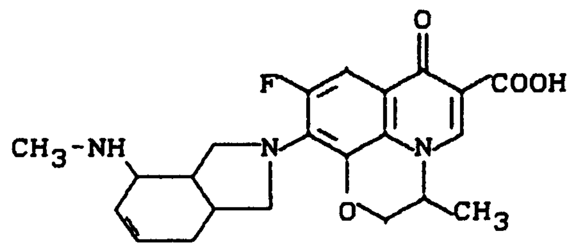

Analog Beispiel 1 wird mit 9,10-Difluor-2,3-dihydro-3-methyl-7-oxo-7H-pyrido[1,2,3-de][1,4]benzoxacin-6-carbonsäure zu 9-Fluor-2,3-dihydro-3-methyl-10-(4-methylamino-1,3,3a,4,7,7a-hexahydro-isoindol-2-yl)-7-oxo-7H-pyrido[1,2,3-de][1,4]benzoxacin-6-carbonsäure vom Schmelzpunkt 207-212° C (unter Zersetzung) umgesetzt.

Beispiel 13

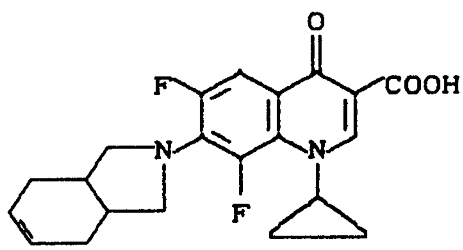

Analog Beispiel 1 erhält man mit 1,3,3a,4,7,7a-Hexahydro-isoindol [J.Org.Chem. 39, 319 (1974)] 1-Cyclopropyl-6,8-difluor-7-(1,3,3a,4,7,7a-hexahydro-isoindol-2-yl)-1,4-dihydro-4-oxo-3-chinoloncarbonsäure vom Schmelzpunkt 269 - 272° C (unter Zersetzung).

Beispiel 14

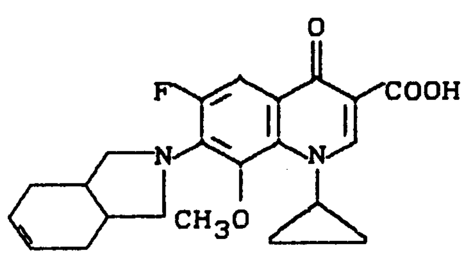

Analog Beispiel 13 erhält man mit 1-Cyclopropyl-6,7-difluor-1,4-dihydro-8-methoxy-4-oxo-3-chinolincarbonsäure 1-Cyclopropyl-6-fluor-7-(1,3,3a,4,7,7a-hexahydro-isoindol-2-yl)-1,4-dihydro-4-oxo-3-chinolincarbonsäure vom Schmelzpunkt 229 - 231° C (unter Zersetzung).

Analog Beispiel 1 erhält man mit den Produkten aus den Beispielen D und E:

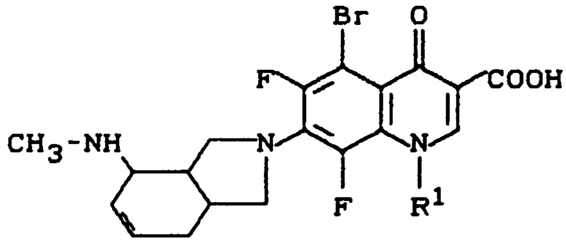

Beispiel 15

($R^1$ = Cyclopropyl): 5-Brom-1-cyclopropyl-6,8-difluor-1,4-dihydro-7-(4-methylamino-1,3,3a,4,7,7a-hexahydroisoindol-2-yl)-4-oxo-3-chinolincarbonsäure,
Schmelzpunkt: 208 - 211° C (unter Zersetzung).

Beispiel 16

($R^1$ = 2,4-Difluorphenyl): 5-Brom-1-(2,4-difluorphenyl)-6,8-difluor-1,4-dihydro-7-(4-methylamino-1,3,3a,4,7,7a-hexahydro-isoindol-2-yl)-4-oxo-3-chinolincarbonsäure.

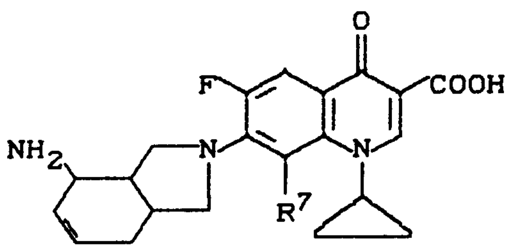

Beispiel 17A: $R^7$ = F
Beispiel 17B: $R^7$ = FxHCl
Beispiel 18A: $R^7$ = Cl
Beispiel 18B: $R^7$ = ClxHCl
Beispiel 19: $R^7$ = H

Beispiel 17

A. Analog Beispiel 1 setzt man mit 4-Amino-1,3,3a,4,7,7a-hexahydro-isoindol zu 7-(4-Amino-1,3,3a,4,7,7a-hexahydro-isoindol-2-yl)-1-cyclopropyl-6,8-difluor-1,4-dihydro-4-oxo-3-chinolincarbonsäure vom Schmelzpunkt 256 -258° C (unter Zersetzung) um.
B. 1,4 g des Betains aus Stufe A werden in 50 ml halbkonzentrierter Salzsäure bei 40° C gelöst, die gelbe Lösung bei 70° C/15 mbar konzentriert, das Kristallisat abgesaugt, mit Ethanol gewaschen und getrocknet.
Ausbeute: 1,3 g (85 % der Theorie) 7-(4-Amino-1,3,3a,4,7,7a-hexahydro-isoindol-2-yl)-1-cyclopropyl-6,8-difluor-1,4-dihydro-4-oxo-3-chinolincarbonsäure
Schmelzpunkt: 272 - 274° C (unter Zersetzung).

Beispiel 18

A. 7-(4-Amino-1,3,3a,4,7,7a-hexahydro-isoindol-2-yl)-8-chlor-1-cyclopropyl-6-fluor-1,4-dihydro-4-oxo-3-chinolincarbonsäure
Schmelzpunkt: 159 - 162° C (unter Zersetzung).
B. 7-(4-Amino-1,3,3a,4,7,7a-hexahydro-isoindol-2-yl)-8-chlor-1-cyclopropyl-6-fluor-1,4-dihydro-4-oxo-3-chinolincarbonsäure-Hydrochlorid
Schmelzpunkt: 241-247° C (unter Zersetzung)

Beispiel 19

7-(4-Amino-1,3,3a,4,7,7a-hexahydro-isoindol-2-yl)-1-cyclopropyl-6-fluor-1,4-dihydro-4-oxo-3-chinolincarbonsäure
Schmelzpunkt: 247 - 249° C (unter Zersetzung) (aus Dimethylformamid).

Beispiel 20

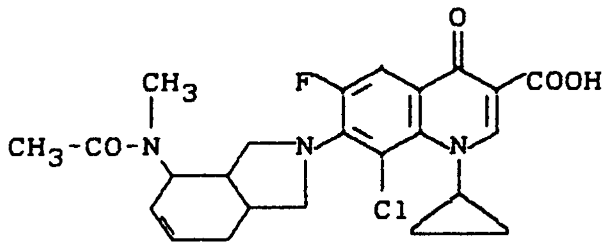

215 mg (0,5 mmol) des Produkts aus Beispiel 3 werden in einer Mischung aus 1,5 ml Dioxan/Wasser (2:1) und 1 ml 1n-Natronlauge gelöst, mit 100 mg Essigsäureanhydrid versetzt und 1 Stunde bei Raumtemperatur gelöst. Der ausgefallene Niederschlag wird abgesaugt, mit Wasser gewaschen und getrocknet. Ausbeute; 194 mg (82 % der Theorie) 7-(N-Acetyl-4-methylamino-1,3,3a,4,7,7a-hexahydro-isoindol-2-yl)-8-chlor-1-cyclopropyl-6-fluor-1,4-dihydro-4-oxo-3-chinolincarbonsäure,
Schmelzpunkt: 217 - 218° C (unter Zersetzung) (aus Acetonitril).

Beispiel 21

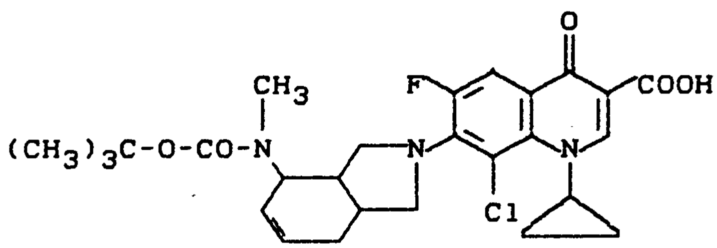

Man setzt analog Beispiel 20 mit Pyrokohlensäure-ditert.-butylester um und erhält 7-(N-tert.-Butoxycarbonyl-4-methylamino-1,3,3a,4,7,7a-hexahydro-isoindol-2-yl)-8-chlor-1-cyclopropyl-6-fluor-1,4-dihydro-4-oxo-3-chinolincarbonsäure vom Schmelzpunkt: 164 - 166° C (unter Zersetzung).

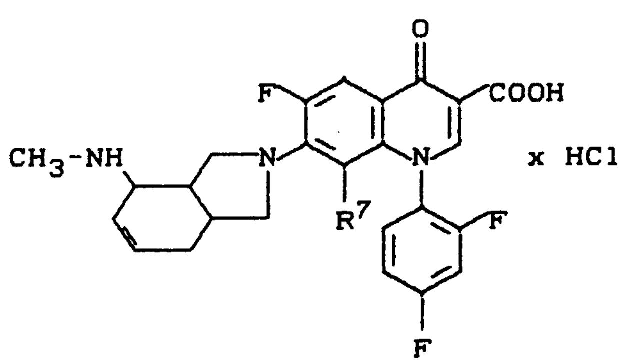

Beispiel 22: $R^7$ = H
Beispiel 23: $R^7$ = F

Beispiel 24: $R^7$ = Cl

Beispiel 22

1,7 g (5 mmol) 1-(2,4-Difluorphenyl)-6,7-difluor-1,4-dihydro-4-oxo-3-chinolincarbonsäure werden in einer Mischung aus 20 ml Acetonitril und 10 ml Dimethylformamid mit 560 mg (5 mmol) 1,4-Diazabicyclo[2.2.2]-octan und 0,9 g (6 mmol) 4-Methylamino-1,3,3a,4,7,7a-hexahydroisoindol versetzt und während 5 Stunden bei 60 - 80° C gerührt. Die Lösung wird mit der doppelten Menge Wasser versetzt und mit 1n-Salzsäure auf pH 7 - 8 eingestellt. Die Mischung bleibt 1 Tag im Kühlschrank stehen, der ausgefallene Niederschlag wird abgesaugt, mit Wasser gewaschen [Rohausbeute: 2 g, Schmelzpunkt: ab 235° C (unter Zersetzung)] und zur Überführung ins Hydrochlorid in einer Mischung aus 2 ml halbkonzentrierter Salzsäure und 7 ml Wasser suspendiert. Man erwärmt auf etwa 30 - 40° C, kühlt auf 0° C ab, saugt das Salz ab und trocknet im Exsikkator.
Ausbeute: 1,4 g (55 % der Theorie) 1-(2,4-Difluorphenyl)-6-fluor-1,4-dihydro-7-(4-methylamino-1,3,3a,4,7,7a-hexahydro-isoindol-2-yl)-4-oxo-3-chinolincarbonsäure-Hydrochlorid,
Schmelzpunkt: ab 285° C (unter Zersetzung)

Beispiel 23

Analog Beispiel 22 erhält man:
1-(2,4-Difluorphenyl)-6,8-difluor-1,4-dihydro-7-(4-methylamino-1,3,3a,4,7,7a-hexahydro-isoindol-2-yl)-4-oxo-3-chinolincarbonsäure-Hydrochlorid,
Schmelzpunkt: ab 230° C (unter Zersetzung)

Beispiel 24

Analog Beispiel 22 erhält man:
8-Chlor-1-(2,4-difluorphenyl)-6-fluor-1,4-dihydro-7-(4-methylamino-1,3,3a,4,7,7a-hexahydro-isoindol-2-yl)-4-oxo-3-chinolincarbonsäure-Hydrochlorid,
Schmelzpunkt: ab 270° C (unter Zersetzung)

Beispiel 25

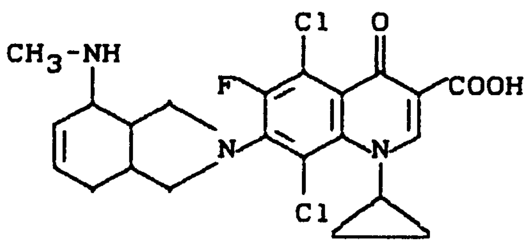

0,67 g (2 mmol) 5,8-Dichlor-1-cyclopropyl-6,7-difluor-1,4-dihydro-4-oxo-3-chinolincarbonsäure, 0,44 g (4 mmol) 1,4-Diazabicyclo[2.2.2]octan und 0,34 g (2,2 mmol) 4-Methylamino-1,3,3a,4,7,7a-hexahydro-isoindol werden in einer Mischung aus 7 ml Acetonitril und 5 ml Dimethylformamid 2 Stunden bei 50° C gerührt. Nach Abkühlung werden 20 ml Wasser zugegeben und mit 10 %iger Salzsäure ein pH von 6 - 7 eingestellt. Nach Einengen im Vakuum wird der Rückstand mit einer Mischung aus Methylenchlorid/Methanol/Wasser (2:4:1) aufgekocht. Nach Abkühlung wird abgesaugt und im Hochvakuum bei 50° C getrocknet.
Ausbeute: 0,59 g (63 % der Theorie) 5,8-Dichlor-1-cyclopropyl-6-fluor-1,4-dihydro-7-(4-methylamino-1,3,3a,4,7,7a-hexahydro-isoindol-2-yl)-4-oxo-3-chinolincarbonsäure
Schmelzpunkt: 162 - 164° C
Analog Beispiel 1 erhält man mit dem Produkt aus Beispiel H:

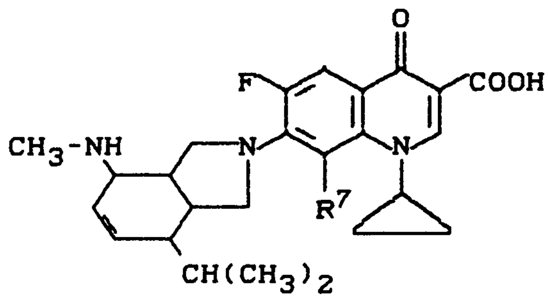

Beispiel 26 ($R^7$ = F)

1-Cyclopropyl-6,8-difluor-1,4-dihydro-7-(7-isopropyl-4-methylamino-1,3,3a,4,7,7a-hexahydro-isoindol-2-yl)-4-oxo-3-chinolincarbonsäure vom Schmelzpunkt 266-271° C (unter Zersetzung) (aus Glykolmonomethylether).

Beispiel 27 ($R^7$ = Cl)

8-Chlor-1-cyclopropyl-6-fluor-1,4-dihydro-7-(7-isopropyl-4-methylamino-1,3,3a,4,7,7a-hexahydro-isoindol-2-yl)-4-oxo-3-chinolincarbonsäure vom Schmelzpunkt 220-223° C (unter Zersetzung) (aus Dimethylformamid).

Analog Beispiel 1 erhält man mit dem Produkt aus Beispiel I:

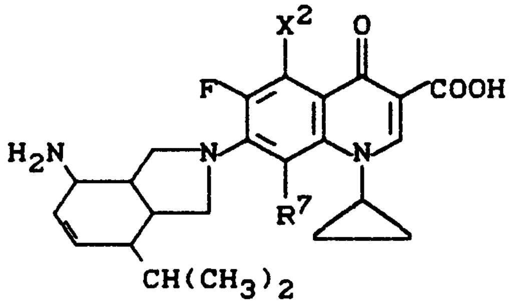

Beispiel 28 ($X^2$ = H, $R^7$ = F)

7-(4-Amino-7-isopropyl-1,3,3a,4,7,7a-hexahydro-isoindol-2-yl)-1-cyclopropyl-6,8-difluor-1,4-dihydro-4-oxo-3-chinolincarbonsäure vom Schmelzpunkt 209-211° C (unter Zersetzung).

Beispiel 29 ($X^2$ = $CH_3$, $R^7$ = H)

7-(4-Amino-7-isopropyl-1,3,3a,4,7,7a-hexahydro-isoindol-2-yl)-1-cyclopropyl-6-fluor-1,4-dihydro-5-methyl-4-oxo-3-chinolincarbonsäure vom Schmelzpunkt 274 bis 275° C (unter Zersetzung). MS: m/e 439 ($M^+$)

Analog Beispiel 1 erhält man mit den Verbindungen aus den Beispielen J, K beziehungsweise L:

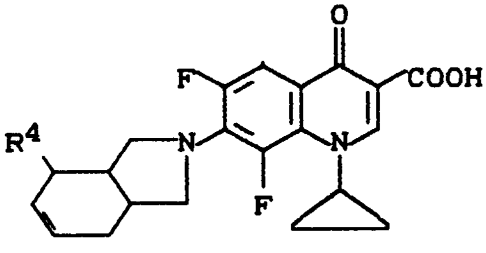

Beispiel 30 ($R^4$ = $CH_2$-OH)

1-Cyclopropyl-6,8-difluor-1,4-dihydro-7-(4-hydroxymethyl-1,3,3a,4,7,7a-hexahydro-isoindol-2-yl)-4-oxo-3-chinolincarbonsäure vom Schmelzpunkt 254-255° C (unter Zersetzung) (aus Dimethylformamid).

Beispiel 31 ($R^4$ = $CH_2$-NH-$CH_3$)

1-Cyclopropyl-6,8-difluor-1,4-dihydro-7-(4-methylaminomethyl-1,3,3a,4,7,7a-hexahydro-isoindol-2-yl)-4-oxo-3-chinolincarbonsäure vom Schmelzpunkt 242-244° C (unter Zersetzung).

Beispiel 32 ($R^4$ = $CH_2$-$NH_2$)

7-(4-Aminomethyl-1,3,3a,4,7,7a-hexahydro-isoindol-2-yl)-1-cyclopropyl-6,8-difluor-1,4-dihydro-4-oxo-3-chinolincarbonsäure.

Analog Beispiel 1 erhält man mit der Verbindung aus Beispiel M:

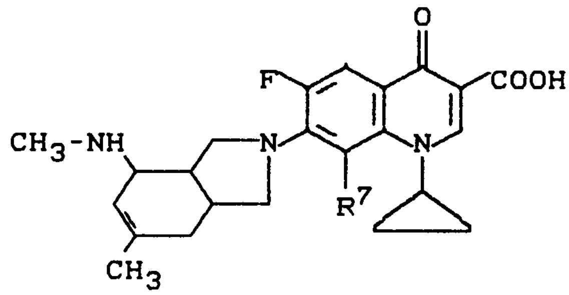

Beispiel 33 ($R^7$ = F)

1-Cyclopropyl-6,8-difluor-1,4-dihydro-7-(6-methyl-4-methylamino-1,3,3a,4,7,7a-hexahydro-isoindol-2-yl)-4-oxo-3-chinolincarbonsäure vom Schmelzpunkt 223-224° C (unter Zersetzung) (aus Glykolmonomethylether)

Beispiel 34 ($R^7$ = Cl)

8-Chlor-1-cyclopropyl-6-fluor-1,4-dihydro-7-(6-methyl-4-methylamino-1,3,3a,4,7,7a-hexahydro-isoindol-2-yl)-4-oxo-3-chinolincarbonsäure vom Schmelzpunkt 198-200° C (unter Zersetzung) (aus Glykolmonomethylether/Dimethylformamid).

Analog Beispiel 1 erhält man mit der Verbindung aus Beispiel N:

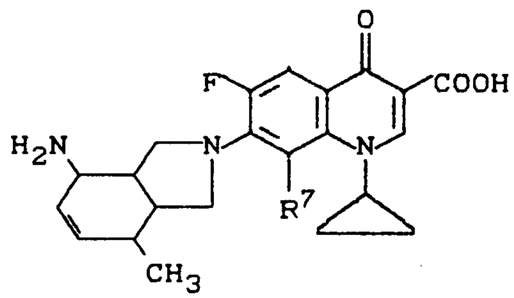

Beispiel 35 ($R^7$ = F)

7-(4-Amino-7-methyl-1,3,3a,4,7,7a-hexahydro-isoindol-2-yl)-1-cyclopropyl-6,8-difluor-1,4-dihydro-4-oxo-3-chinolincarbonsäure, Schmelzpunkt: 196 bis 198° C (unter Zersetzung) (aus Glykolmonomethylether).

Beispiel 36 ($R^7$ = Cl)

7-(4-Amino-7-methyl-1,3,3a,4,7,7a-hexahydro-isoindol-2-yl)-8-chlor-1-cyclopropyl-6-fluor-1,4-dihydro-4-oxo-3-chinolincarbonsäure, Schmelzpunkt: 124 bis 129° C (unter Zersetzung) (aus Glykolmonomethylether).

Beispiel 37 ($R^7$ = H)

7-(4-Amino-7-methyl-1,3,3a,4,7,7a-hexahydro-isoindol-2-yl)-1-cyclopropyl-6-fluor-1,4-dihydro-4-oxo-3-chinolincarbonsäure, Schmelzpunkt: 223 bis 225° C (unter Zersetzung) (aus Glykolmonomethylether).

Beispiel 38

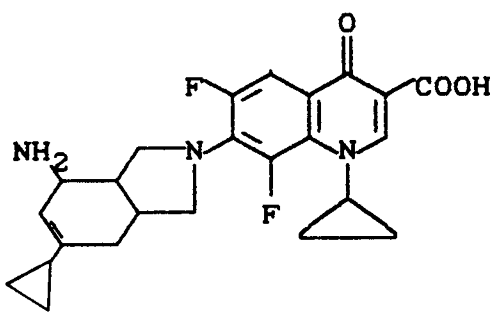

7-(4-Amino-7-cyclopropyl-1,3,3a,4,7,7a-hexahydro-isoindol-2-yl)-1-cyclopropyl-6,7-difluor-1,4-dihydro-4-oxo-3-chinolincarbonsäure wird analog Beispiel 1 mit der Verbindung aus Beispiel O hergestellt. Schmelzpunkt; 236-237° C (unter Zersetzung) (aus Glykolmonomethylether).

Beispiel 39

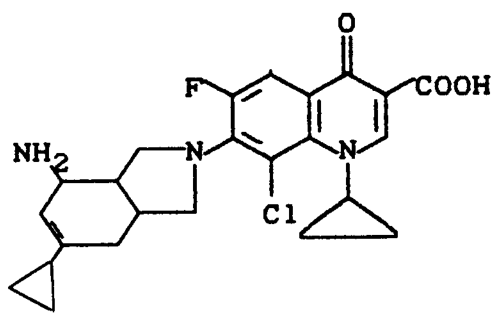

7-(4-Amino-7-cyclopropyl-1,3,3a,4,7,7a-hexahydro-isoindol-2-yl)-8-chlor-1-cyclopropyl-6-fluor-1,4-dihydro-4-oxo-3-chinolincarbonsäure wird analog Beispiel 1 durch Umsetzung von 8-Chlor-1-cyclopropyl-6,7-difluor-1,4-dihydro-4-oxo-3-chinolincarbonsäure mit der Verbindung aus Beispiel O hergestellt. Schmelzpunkt: 177-179° C (unter Zersetzung) (aus Glykolmonomethylether.

## Patentansprüche

**1.** Chinolon- und Naphthyridoncarbonsäure-Derivate der Formel (I)

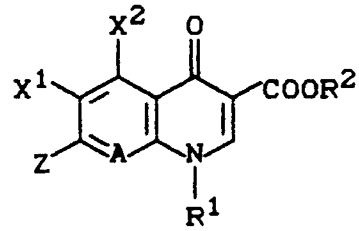

(I),

in welcher

- $X^1$ für Halogen,
- $X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,
- $R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
- $R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,
- Z für einen Rest der Struktur

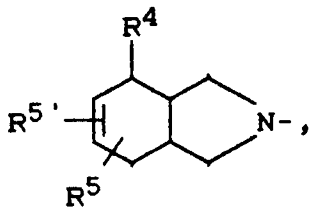

steht, worin

- $R^4$ für Wasserstoff, Hydroxy,

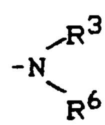

,

Hydroxymethyl oder

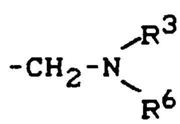

steht, wobei

- $R^3$ Wasserstoff, gegebenenfalls durch Hydroxy substituiertes $C_1$-$C_3$-Alkyl, Alkoxycarbonyl mit 1 bis 4 C-Atomen im Alkoxyteil oder $C_1$-$C_3$-Acyl und
- $R^6$ Wasserstoff oder Methyl bedeutet,
- $R^5$ für Wasserstoff, $C_1$-$C_3$-Alkyl oder Cyclopropyl,
- $R^{5'}$ für Wasserstoff oder Methyl und
- A für N oder C-$R^7$ steht, worin
- $R^7$ für H, Halogen, Methyl, Hydroxy oder Methoxy steht oder auch gemeinsam mit $R^1$ eine Brücke der Struktur

$-O-CH_2-\overset{*}{C}H-CH_3$, $-S-CH_2-\overset{*}{C}H-CH_3$ oder

$-CH_2-CH_2-\overset{*}{C}H-CH_3$

bilden kann,

und deren pharmazeutisch verwendbaren Hydrate und Säureadditionssalze sowie Alkali-, Erdalkali-, Silber- und Guanidiniumsalze.

**2.** Verbindungen gemaß Anspruch 1 der Formel (I),

in welcher
$X^1$ für Fluor,
$X^2$ für Wasserstoff, Amino, Methylamino, Hydroxy, Methoxy, Fluor, Chlor, Brom oder Methyl,
$R^1$ für Alkyl mit 1 bis 3 Kohlenstoffatomen, Vinyl, Cycloalkyl mit 3 bis 4 Kohlenstoffatomen, 2-Fluorethyl, gegebenenfalls durch 1 oder 2 Fluoratome substituiertes Phenyl,
$R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,
Z für einen Rest der Struktur

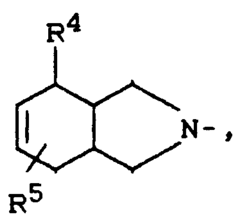

steht, worin
$R^4$ für Wasserstoff, Hydroxy oder

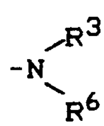

steht, wobei
$R^3$ Wasserstoff, gegebenenfalls durch Hydroxy substituiertes $C_1$-$C_2$-Alkyl, Alkoxycarbonyl mit 1 bis 4 C-Atomen im Alkoxyteil oder $C_1$-$C_3$-Acyl und
$R^6$ Wasserstoff oder Methyl bedeutet,
$R^5$ für Wasserstoff oder Methyl und
A für N oder C-$R^7$ steht, worin
$R^7$ für H, Fluor, Chlor, Brom, Methyl oder Methoxy steht oder auch gemeinsam mit $R^1$ eine Brücke der Struktur

$-O-CH_2-\overset{*}{C}H-CH_3$,

bilden kann

und deren pharmazeutisch verwendbare Hydrate und Säureadditionssalze sowie Alkali-, Erdalkali-, Silber- und Guanidiniumsalze.

**3.** Verbindungen gemäß Anspruch 1 der Formel (I),

in welcher
$X^1$ für Fluor,
$X^2$ für Wasserstoff, Amino, Fluor, Chlor oder Brom,
$R^1$ für Alkyl mit 1 bis 2 Kohlenstoffatomen, Cyclopropyl, gegebenenfalls durch 1 oder 2 Fluoratome substituiertes Phenyl,
$R^2$ für Wasserstoff oder Alkyl mit 1 bis 2 Kohlenstoffatomen,
Z für einen Rest der Struktur

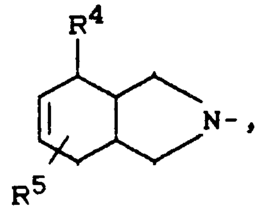

steht, worin
$R^4$ für Wasserstoff, Hydroxy oder

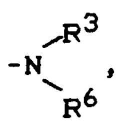

steht, wobei
$R^3$ Wasserstoff, Methyl, Alkoxycarbonyl mit 1 bis 4 C-Atomen im Alkoxyteil oder $C_1$-$C_3$-Acyl und
$R^6$ Wasserstoff oder Methyl bedeutet,
$R^5$ für Wasserstoff oder Methyl und
A für N oder C-$R^7$ steht, worin
$R^7$ für H, Fluor, Chlor oder Methoxy steht oder auch gemeinsam mit $R^1$ eine Brücke der Struktur

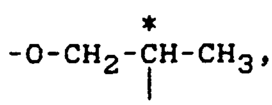

bilden kann,
und deren pharmazeutisch verwendbaren Hydrate und Säureadditionssalze sowie Alkali-, Erdalkali-, Silber- und Guanidiniumsalze.

**4.** 1-Cyclopropyl-6,8-difluor-1,4-dihydro-7-(4-methyl-amino-1,3,3a,4,7,7a-hexahydro-isoindol-2-yl)-4-oxo-3-chinolincarbonsäure.

**5.** 8-Chlor-1-cyclopropyl-6-fluor-1,4-dihydro-7-(4-methylamino-1,3,3a,4,7,7a-hexyhydro-isoindol-2-yl)-4-oxo-3-chinolincarbonsäure.

**6.** 8-Chlor-1-cyclopropyl-6-fluor-1,4-dihydro-7-(7-methyl-4-methylamino-1,3,3a,4,7,7a-hexahydroisoindol-2-yl)-4-oxo-3-chinolincarbonsäure.

**7.** 7-(4-Amino-1,3,3a,4,7,7a-hexahydro-isoindol-2-yl)-1-cyclopropyl-6,8-difluor-1,4-dihydro-4-oxo-3-chinolincarbonsäure.

**8.** 7-(4-Amino-1,3,3a,4,7,7a-hexahydro-isoindol-2-yl)-8-chlor-1-cyclopropyl-6-fluor-1,4-dihydro-4-oxo-3-chinolincarbonsäure.

**9.** 7-(4-Amino-1,3,3a,4,7,7a-hexahydro-isoindol-2-yl)-1-cyclopropyl-6-fluor-1,4-dihydro-4-oxo-3-chinolincarbonsäure.

**10.** 5-Brom-1-cyclopropyl-6,7,8-trifluor-1,4-dihydro-4-oxo-3-chinolincarbonsäure.

**11.** 5-Brom-1-(2,4-difluorphenyl)-6,7,8-trifluor-1,4-dihydro-4-oxo-3-chinolincarbonsäure.

**12.** 5,8-Dichlor-1-cyclopropyl-6,7-difluor-4-oxo-3-chinolincarbonsäure.

**13.** 5,8-Dichlor-1-(2,4-difluorphenyl)-6,7-difluor-1,4-dihydro-4-oxo-3-chinolincarbonsäure.

**14.** Substituierte 4-Amino-1,3,3a,4,7,7a-hexahydroisoindole der Formel

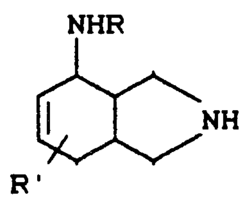

in der R und R' Wasserstoff oder Methyl bedeuten.

**15.** Verfahren zur Herstellung von Verbindungen gemäß Anspruch 1 der Formel (I), dadurch gekennzeichnet, daß man Verbindungen der Formel (II)

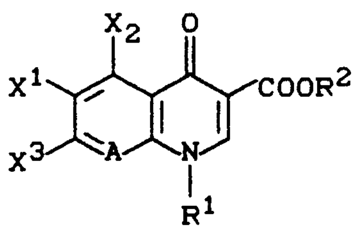

(II),

in welcher

A, $R^1$, $R^2$, $X^1$ und $X^2$ die oben angegebene Bedeutung haben und
$X^3$ für Halogen, insbesondere Fluor oder Chlor steht,

mit Verbindungen der Formel (III)

Z-H (III)

in welcher
Z die oben angegebene Bedeutung hat,
gegebenenfalls in Gegenwart von Säurefängern umsetzt.

**16.** Arzneimittel enthaltend Verbindungen gemäß Ansprüchen 1 bis 9.

**17.** Verwendung von Verbindungen gemäß Ansprüchen 1 bis 9 bei der Herstellung von Arzneimitteln.

Europäisches Patentamt

# EUROPÄISCHER RECHERCHENBERICHT

Nummer der Anmeldung

EP 92 10 9742

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 343 560 (WAKUNAGA SEIYAKU KABUSHIKI KAISHA)<br>* Ansprüche * | 1,16 | C07D401/04<br>C07D209/44<br>C07D215/56<br>A61K31/47 |
| A | EP-A-0 429 304 (SHIONOGI SEIYAKU KABUSHIKI KAISHA)<br>* Ansprüche * | 1,16 | |
| A | EP-A-0 343 524 (SHIONOGI SEIYAKU KABUSHIKI KAISHA)<br>* Ansprüche * | 1,16 | |
| Y | EP-A-0 391 132 (BAYER AG)<br>* Seite 53, Zeile 1-15 * | 10-13 | |
| Y | EP-A-0 247 464 (FUJISAWA PHARMACEUTICAL CO., LTD.)<br>* Seite 3, Verbindung III * | 10-13 | |
| Y | JOURNAL OF MEDICINAL CHEMISTRY.<br>Bd. 33, 1990, WASHINGTON US<br>Seiten 1645 - 1656;<br>TERUYUKI MIYAMOTO ET AL.: 'Synthesis and structure-activity relationships of 5-substituted 6,8-difluoroquinolones, including sparfloxacin, a new quinolone antibacterial agent with improved potency.'<br>* Seite 1647, Verbindungen 29, 30 * | 10-13 | RECHERCHIERTE SACHGEBIETE (Int. Cl.5)<br>C07D |

Der vorliegende Recherchenbericht wurde für alle Patentansprüche erstellt

| Recherchenort | Abschlußdatum der Recherche | Prüfer |
|---|---|---|
| DEN HAAG | 21 JULI 1992 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)